# EUROPEAN PATENT SPECIFICATION

(11) **EP 4 566 554 B1**
(45) Date of publication and mention of the grant of the patent: **28.10.2026**
(21) Application number: 25159320.8
(22) Date of filing: 14.08.2015
(51) Int. Cl.: A61B 17/34, A61B 17/42

(54) **NATURAL ORIFICE SURGERY SYSTEM**
CHIRURGISCHES SYSTEM FÜR NATÜRLICHE ÖFFNUNG
SYSTÈME DE CHIRURGIE D'ORIFICE NATUREL

(30) Priority: 15.08.2014 US 201462038082 P
(43) Date of publication of application: 11.06.2025
(60) Divisional application: 26206025.4
(62) Document number(s) of the earlier application(s) in accordance with Art. 76 EPC: 22188302.8 / 4 101 405; 19152405.7 / 3 498 204; 15753858.8 / 3 179 934
(73) Proprietor: Applied Medical Resources Corporation, Rancho Santa Margarita, CA 92688 (US)
(72) Inventor: WACHLI, Serene, Rancho Santa Margarita, 92688 (US); BRESLIN, Tracy, Rancho Santa Margarita, 92688 (US); SHEEHAN, Alexander, Rancho Santa Margarita, 92688 (US); POULSEN, Nikolai, Rancho Santa Margarita, 92688 (US)
(74) Representative: Dolleymores

(56) References cited:
- WO-A2-2009/117435
- US-A1- 2011 144 443
- US-A1- 2013 245 381

## Description

### BACKGROUND

### Technical Field

This application is directed to surgical access port system and more particularly, to surgical access port adapted for use with a cap, that is useful in natural orifice single-port surgical procedures and which is particularly useful in vaginal surgical procedures.

### Description of the Related Art

Access devices are commonly used in surgery to facilitate the introduction of various surgical instruments into natural biological vessels, conduits, orifices, cavities, and other interior regions of the body. These access devices include, for example, devices that facilitate the introduction of a needle into a vessel, and trocars that facilitate the introduction of laparoscopic instruments into the abdomen of the body. Examples of such devices are disclosed in patent documents bearing publication numbers US 2013/245381 A1, WO 2009/117435 A2 and US 2011/144443 A1.

Some of these access devices are introduced into regions that include a fluid or gas under pressure. In the case of a needle access device, the pressure may be from a liquid, such as blood. In the case of a trocar, the pressure may be from a gas, such as an insufflation gas. In either case, it is desirable to provide for the introduction of the surgical instrument into the cavity without permitting the escape of the pressurized fluid or gas.

In the case of trocars, a cannula at the distal end of the trocar is typically connected to a seal housing at the proximal end of the trocar. Together the cannula and housing form a working channel through which various instruments can be inserted to access the cavity. Seal mechanisms are commonly disposed in the housing and include a septum valve that seals the working channel when an instrument is in place, and a zero closure valve that seals the working channel when the instrument is removed.

Current surgical access ports allow for single instrument access through each port, or allow for multiple instrument access through a rigid cannula. Some devices, such as transanal endoscopic microsurgery (TEMS) units require that the device be attached to the surgical table to support the weight of the device, as well as to locate the position of the device respective to the patient. These devices do not provide flexibility to the surgeon in selecting instrument size, and they restrict instrument movement with their rigid cannulas. Additionally, surgeons are performing laparoscopic surgical procedures through a single or a limited number of access ports. The procedures may be performed through a single two (2) centimeter incision at the umbilicus, or in certain cases, trans-vaginally or trans-anally. What is needed is a system that meets the needs of these new procedures, facilitating more flexible movement of laparoscopic instruments through a single or limited number of ports while preventing the escape of pressured fluids or gasses and permitting large specimen removal. What is particularly needed is an access system that can be deployed into a natural body cavity, such as the vagina, providing a sealed system for insufflation and a platform for the introduction of multiple surgical instruments of varying sizes, while maintaining sufficient stability for anchoring the system within the cavity without damaging the body cavity wall.

### SUMMARY OF THE INVENTION

The invention is directed to a surgical access port system, as recited in Claim 1, adapted for performing surgical procedures at a natural orifice.

### BRIEF DESCRIPTION OF THE DRAWINGS

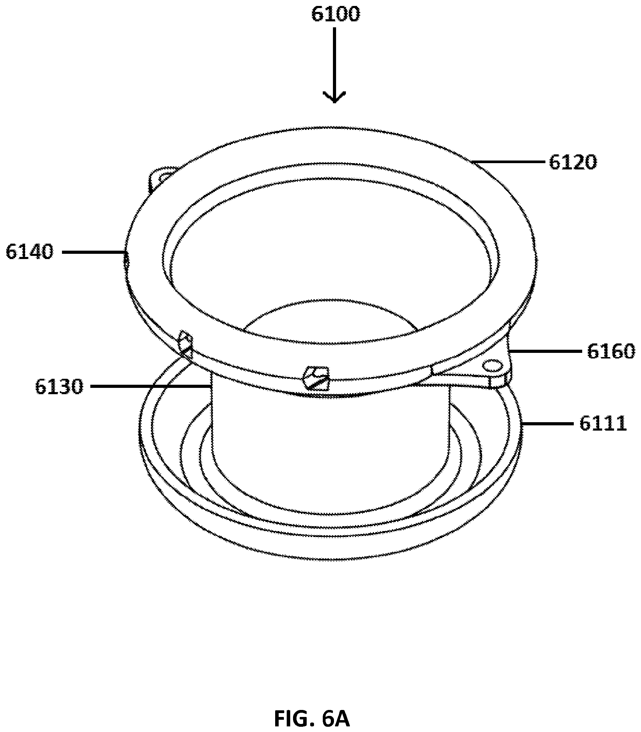

The invention will now be described with reference to the figures, where surgical access port systems are illustrated together with various components of those systems. Many of these are relevant to the understanding of the present invention, with figures 6A to 6D depicting retractors which, when used in such systems, will result in those systems being in accordance with the present invention.
FIG. **1** is a side view of a patient in surgery illustrating an access device positioned on the abdomen and in use.
FIG. **2** is a cross-sectional side view illustrating an access device, with the wound retractor retracting the vagina of a patient, and the gel cap sealing the opening of the wound retractor.
FIG. 3 is a front view illustrating an access device deployed and in use at the mouth of the patient.
FIG. **4** is a top view illustrating a patient in the prone position with an access device deployed and in use at the anus of the patient.
FIG. **5A** is a picture of the components of an access device system useful for natural orifice surgery; FIG. **5B** is a perspective view of the retractor of the access device system FIG. 5A.
FIG. **6A** is a perspective view of an of a natural orifice retractor having an occlusion flange suitable as the retractor of the access device system of FIG. **5A****,** then in accordance with the present invention. FIG. **6B** is a side cut away of the natural orifice retractor of FIG. 6A.
FIG. **6C** is a side view of an embodiment of a natural orifice retractor having a compression flange. FIG. **6D** is a side cut away of the natural orifice retractor of FIG. 6C.
FIG. **6E** is a perspective view of an embodiment of a natural orifice retractor having a bolster cone.
FIG. **6F** is a side view of an embodiment of a natural orifice retractor having a mechanical balloon, shown in an uncompressed state. FIG. **6G** is a side view of the natural orifice retractor of FIG. **6F****,** shown in a compressed, or expanded, state.
FIG. **6H** is a perspective view of an embodiment of a natural orifice retractor having a balloon.
FIG. **6I** is a perspective view of an embodiment of a natural orifice retractor having a saddle balloon.
FIG. **6J** is a perspective view of an alternative embodiment of a retractor having an inflatable member.
FIG. **6K** is a cutaway side view showing the check valve and channel disposed in the tubular body of the retractor.
FIG. **6L** is a perspective view of an embodiment of a natural orifice retractor having a balloon and a bolster.
FIG. **6M** is a perspective view of an embodiment of a natural orifice retractor adapted to provide retraction and access without insufflation, shown in an open configuration. FIG. **6N** is a cross-sectional view of the embodiment of a natural orifice retractor of FIG. **6M****.** FIG. **6O** is a close-up side view of the screw threads disposed around the retaining sleeve and the tubular body of the retractor. FIG. **6P** is a cross-sectional view of the embodiment of FIG. **6M****,** shown in a closed configuration.
FIG. **6Q** is a close-up side view of the ratcheting mechanism for attaching the retaining sleeve to the tubular body of the retractor.
FIG. **7A** is a perspective view of an obturator adapted to facilitate introduction of a natural orifice retractor into a body orifice such as a vagina. FIG. **7B** is a side view of the obturator of FIG. **7A****.**
FIG. **7C** is a perspective view of an obturator having a straight shaft piece, adapted to facilitate introduction of a natural orifice retractor into a body orifice such as a vagina. FIG. **7D** is a perspective view of a retractor disposed on the obturator of FIG. **7C****.**
FIG. **7E** is a perspective view of an obturator, modified with an indent to provide clearance for the inflation port shown in FIG. **6I** and **6J** and adapted to facilitate introduction of a natural orifice retractor into a body orifice such as a vagina.
FIG. **8A** is a side view of the natural orifice access device of FIG. **5A****.** FIG. **8B** is a top view of the natural orifice access device illustrated in FIG. **5A****.** FIG. **8C** is a perspective view of the natural orifice access device illustrated in FIG. **5A****.**
FIG. **9A** is a perspective view of an embodiment of a natural orifice access device including a cap having a plurality of ports extending there through.
FIG. **9B** is an exploded view of an embodiment of a port and optional obturator, which is a component of some embodiments of the access device system.
FIG. **10A** is a top perspective view of an embodiment of a gel cap. FIG. **10B** is a bottom view of an embodiment of a cap ring.
FIG. **11A** is a top view of an embodiment of a gel cap comprising a plurality of access ports embedded in the gel pad. FIG. **11B** is a top perspective view of the gel cap illustrated in FIG. **11A****.** FIG. **11C** is a bottom perspective view of the gel cap illustrated in FIG. **11A****.**
FIG. **11D** is a top perspective view of the gel cap illustrated in FIG. **11A** with instruments inserted through two of the access ports. FIG. **11E** is a bottom perspective view of the gel cap and instruments illustrated in FIG. **11D****.** FIG. **11F** is a side view of the gel cap and instruments illustrated in FIG. **11D****.**
FIG. **11G** is a top perspective view of an embodiment of gel cap comprising a fixed camera or laparoscope port.
FIG. **12** is a cutaway perspective view of an embodiment of an access device system comprising a gel cap that snap fits to a retractor.
FIG. **13** is an exploded view of an embodiment of a trocar.
FIGS. **14A** and **14B** are side views of an embodiment of a trocar comprising a fixation cannula in an insertion configuration and a fixation configuration, respectively.
FIG. **15** is a side view of another embodiment of a trocar comprising a fixation cannula.
FIG. **16A** is a side view of another embodiment of a trocar comprising a fixation cannula. FIG. **16B** is a perspective view of an embodiment of a bolster suitable for use with the trocar illustrated in FIG. **16A****.**
FIG. **17A** is a side view of another embodiment of a trocar comprising a fixation cannula. FIG. **17B** is a perspective view of an embodiment of a bolster suitable for use with the trocar illustrated in FIG. **17A****.**

Similar components have similar reference numbers throughout.

### DETAILED DESCRIPTION OF CERTAIN EMBODIMENTS

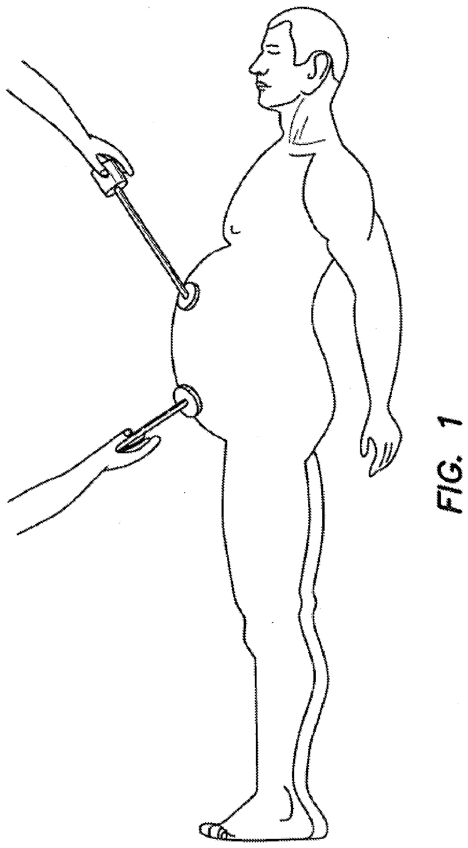

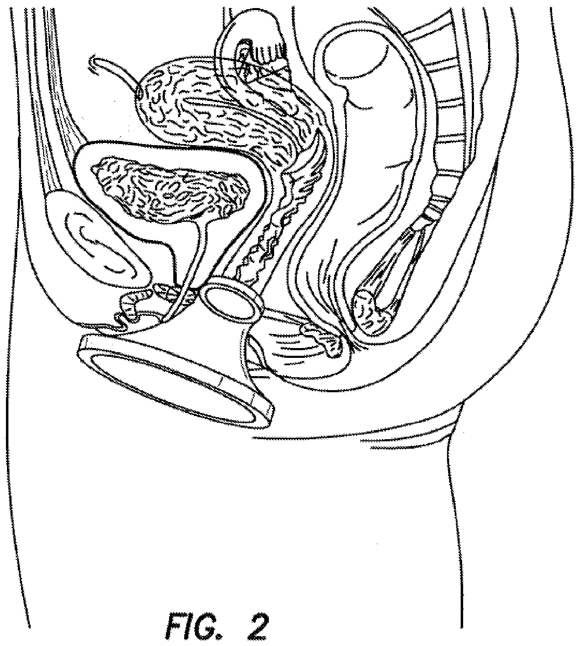

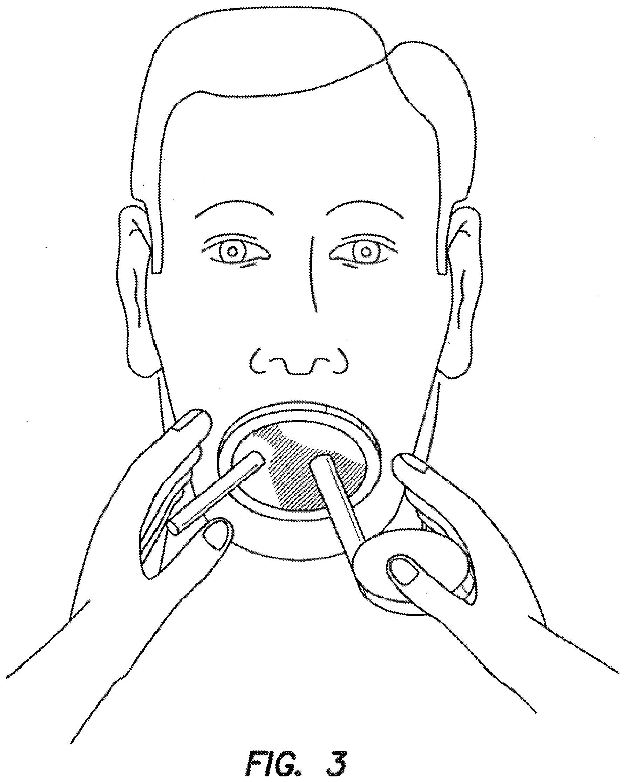

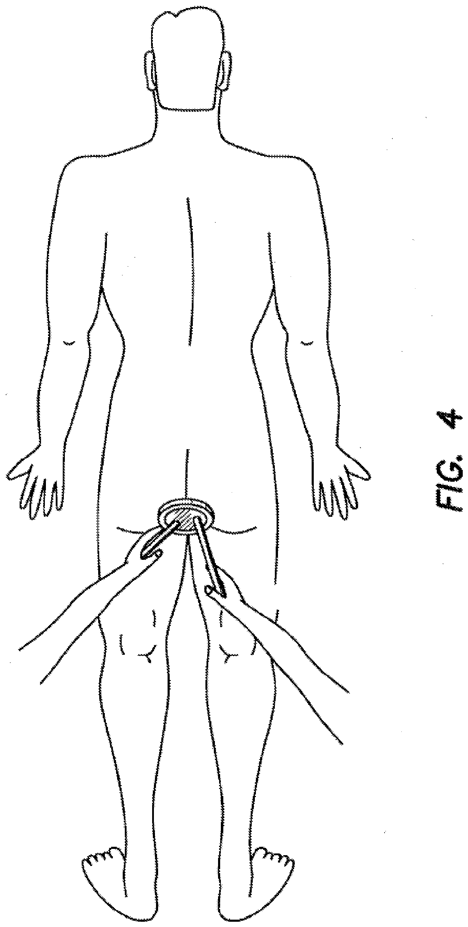

Embodiments of a surgical instrument access device system are useful, for example, for single incision, single port, and/or limited port laparoscopic surgical procedures, for example, abdominal (FIG. **1****),** transvaginal (FIG. **2****),** transoral (FIG. **3****),** and transanal (FIG. **4****)** procedures. Various surgical instrument access devices are described in U.S. Patent Application Publication No. 2009/0187079 and U.S. Patent No. 7,727,146.

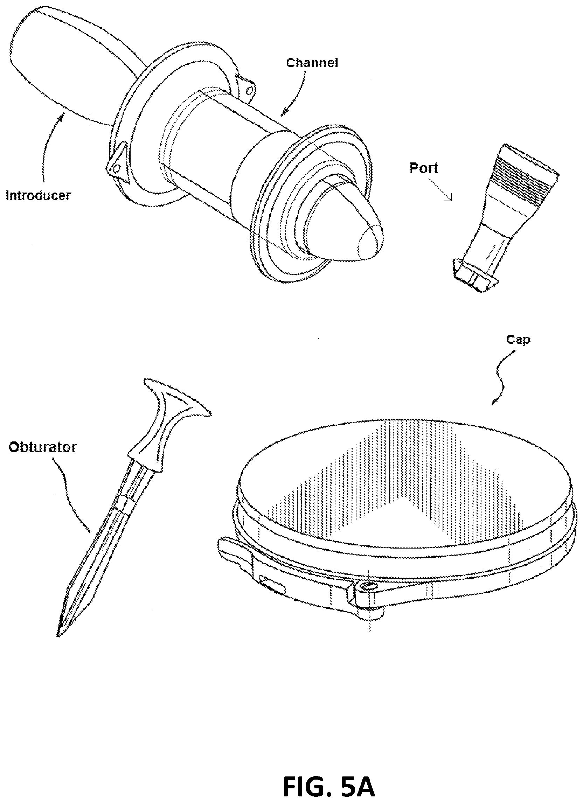

FIG. **5A** shows an embodiment of an access device system comprising a retractor **6100,** an introducer **6400** shown inserted into the retractor as if for placement in a body orifice, a cap **6200,** one or more ports **6310,** and an obturator **6600,** which is useful in single port and/or limited port procedures. The retractor **6100** is placed and/or positioned into, across, and/or through a surgical incision and/or body orifice to enlarge, reshape, and/or isolate the incision or body orifice. The cap **6200** provides an artificial body wall through which instruments access the interior of a patient's body, for example, a body cavity, through ports **6310.** The retractor **6100** may be introduced into an orifice using the introducer **6400;** ports **6310** may be placed through the gel cap using obturator **6600.** The components of the access device comprise any suitable biologically compatible materials.

With the gel cap **6200** attached to the retractor **6100,** the access device system allows the user to insufflate the orifice, such as the vaginal canal. The distention of the canal caused by the insufflation provides greater visualization of the anatomy (when compared, for example, to traditional vaginal hysterectomy) and removes the need for the use of rigid mechanical retractors which may cause damage to soft tissues. The gel cap may be detached at any point during the surgical procedure to allow for specimen removal.

The retractor **6100** is made of a semi-pliable thermoplastic elastomer or thermoset polymer. For use in vaginal procedures, a length of approximately 4 cm to 7 cm and a diameter of approximately 3 cm to 5 cm will suit a range of anatomies. The retractor retracts and occludes the vagina. Suture ties **6160** at proximal end **6152** and/or a flange **6110** along the tubular body may help prevent the retractor from dislodging once placed. The gel cap **6200** may be attached with the aid of a lever **6135** which locks under the proximal ring **6120** of the retractor **6100.** This creates a pressure resistant seal and insufflation is possible through insufflation ports **6145** on the gel cap. Smoke evacuation is possible through the port not being used for insufflation. Instrument ports **6310** may be placed in the gel cap **6200** to allow for the use of various laparoscopic instruments. The gel cap may then be detached and specimens may be removed through the retractor **6100.**

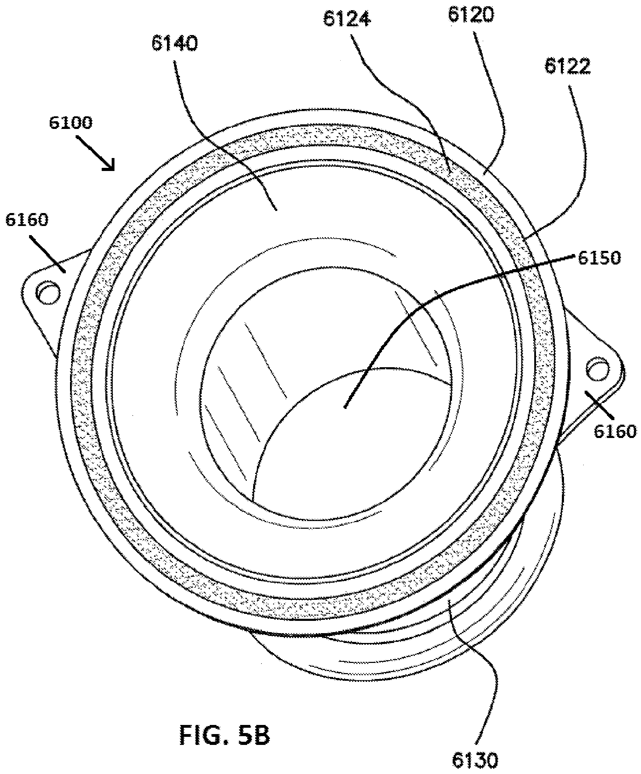

Turning to FIG. **5B****,** the outer ring **6120** is proximal a funnel section **6140.** In the illustrated embodiment, the outer ring **6120** has a substantially circular footprint. As further discussed herein, the outer ring **6120** can be sized and configured to sealingly couple to a cap or other access device thereon. In some embodiments, one or more suture points **6160** can be disposed on the retractor **6100** adjacent the outer ring **6120.** Two suture points **6160** are generally diametrically opposed relative to the generally circular profile of the outer ring **6120.** In other embodiments, the retractor can include more or fewer than two suture points disposed of various locations relative to the outer ring **6120.**

With continued reference to FIG. **5B****,** the tubular body **6130** has a generally circular profile defining a generally cylindrical passage **6150.** The generally cylindrical passage is desirably large enough to accommodate more than one laparoscopic instrument there through such that a single natural orifice access device can be used to provide access for multiple surgical instruments in a body cavity. Moreover, generally cylindrical passage is desirably large enough such that multiple surgical instruments positioned there through can be translated or pivoted relative to one another, allowing a surgeon to manipulate the instruments as desired during a surgical procedure. The generally cylindrical passage extends between a proximal end **6152** of the retractor **6100** adjacent the outer ring **6120** to a distal end **6154** of the retractor **6100** adjacent the flange **6110.** In FIGs **5A** and **5B****,** the tubular body **6130** has a circular cross-section. Some embodiments of the tubular body **6130** comprise one or more coatings that provide additional functionality, for example, an anti-microbial coating.

In the illustrated embodiment, the funnel segment **6140** provides a diametric reduction between the relatively large diameter of the outer ring **6120,** which is sized and configured to be removably coupled to an access device such as a cap, and the relatively smaller diameter of the passage **6150,** which is sized to fit within a natural orifice with minimal distention of the orifice. The funnel segment **6140** has an inner surface which can provide a bearing surface for an obturator or introducer used to advance to the retractor **6100** into a body cavity. In some embodiments, the funnel segment can have a substantially linear taper between the relatively large diameter and the relatively smaller diameter such that the inner surface is a frustoconical segment. In other embodiments, the funnel segment **5** can have a curved profile between the relatively large diameter and the relatively smaller diameter. In some embodiments, there is no funnel section at all, as where the tubular body connects directly to the outer ring.

It can be desirable that the outer ring **6120** is relatively stiff compared with the relatively flexible tubular body **6130** of the retractor **6100** so that the outer ring **6120** can sealingly engage an access device such as a cap. With reference to FIG. **5B****,** a perspective view of the retractor is illustrated with a partial cutaway of the outer ring **6120.** In the illustrated embodiment, the outer ring **6120** includes an annular groove **6122** formed therein in which a reinforcing member **6124** is disposed. In some embodiments, the reinforcing member **6124** can comprise a metallic member such as a wire formed into a ring shape. For example, in some embodiments the reinforcing member **6124** can comprise a stainless steel ring positioned within the groove **6122** during manufacture of the retractor **6100.** In other embodiments, the reinforcing number **6124** can comprise an injectable nonmetallic member. For example, in some embodiments, a glass filled polymer or polycarbonate material can be injected into the groove **6122** during manufacture of the retractor **6100.**

While the illustrated embodiments of retractor **6100** include a reinforcing member to enhance the rigidity of the outer ring **6120,** in other embodiments, the retractor **6100** can be formed in a multiple-shot molding process. For example, in some embodiments, an inner segment of the retractor defined by the tubular body **6130** and the flange **6110** is formed in one molding operation from a flexible material, and an outer segment of the retractor **6100** defined by the funnel segment **6140** and the outer ring **6120** is formed in another molding operation from a relatively rigid material such as a polycarbonate material or other suitable material.

With continued reference to FIG. **5B****,** the illustrated embodiment includes a continuous generally annular groove. In other embodiments, a plurality of noncontiguous recesses can each receive one of a plurality of reinforcing members. Moreover, in some embodiments, the outer ring can include two or more concentric generally annular grooves, which each receive a corresponding reinforcing member.

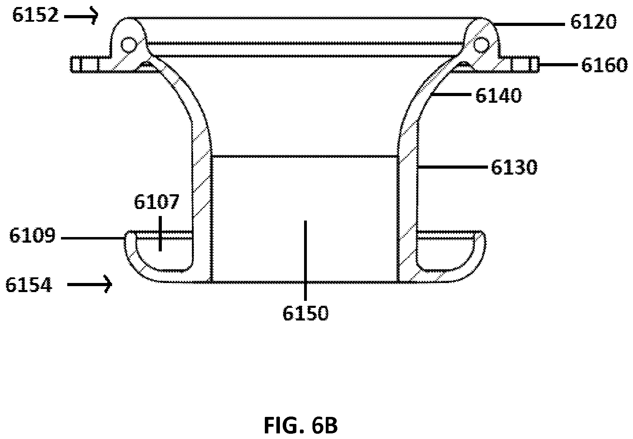

A retractor **6100** of a surgical access port system in accordance with the present invention is illustrated in FIG. **6A** and FIG. **6B****.** This is adapted for use in a transvaginal surgical procedure. The retractor **6100** comprises an occlusion flange **6111,** an outer or proximal ring **6120,** and a tubular body **6130** and a funnel segment **6140** extending between and coupling the occlusion flange **6111** and the outer ring **6120.** The occlusion flange **6111** is semi-rigid, with a diameter from approximately 5.08 cm (2 inches) to 10.16 cm (4 inches). When inserted into the vagina, the flange is under compression from the walls of the vaginal canal, creating a seal to aid in the maintenance of insufflation. The occlusion flange is "cup-shaped" with a circular ridge **6109** circumscribing a channel **6107,** which provides sufficient flexibility to allow the flange **6111** to flex to accommodate different anatomies while still maintaining a seal.

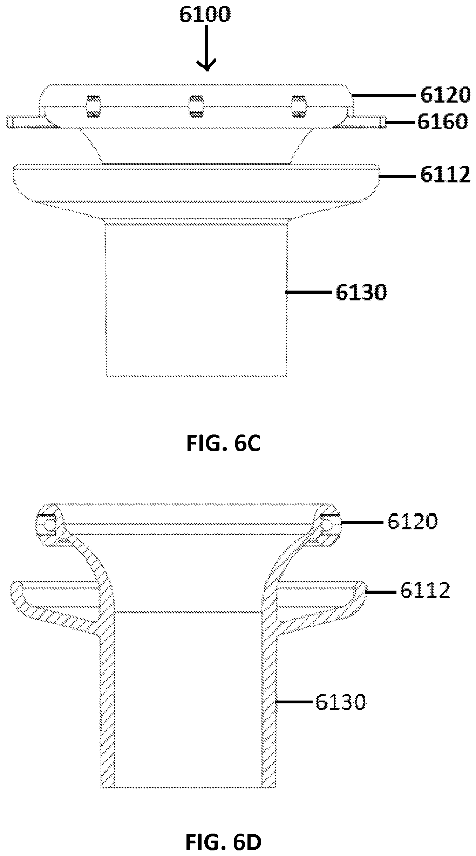

With reference to FIG. **6C****,** a side view of a retractor **6100** for use in the natural orifice access device system having a different flange embodiment is shown. In this embodiment, the retractor **6100** is shown with a compression flange **6112.** The compression flange **6112** is approximately 5.08 cm (2 inches) to 10.16 cm (4 inches) in diameter and is disposed around the retractor distal to the outer or proximal ring **6120.** The distance between the compression flange and the outer ring is designed to be less than the thickness of the tissues comprising the natural orifice opening. In the case of the vaginal opening, the distance is approximately 2.54 mm (0.1 inches) to 12.7 mm (0.5 inches). This creates a compressive force on the tissue between the flange and outer ring of the retractor. This force and the "cup-shaped" geometry of the flange (see FIG. **6D****)** occlude the vagina and serve to prevent dislodging. Suture ties **6160** may be used to mitigate dislodging.

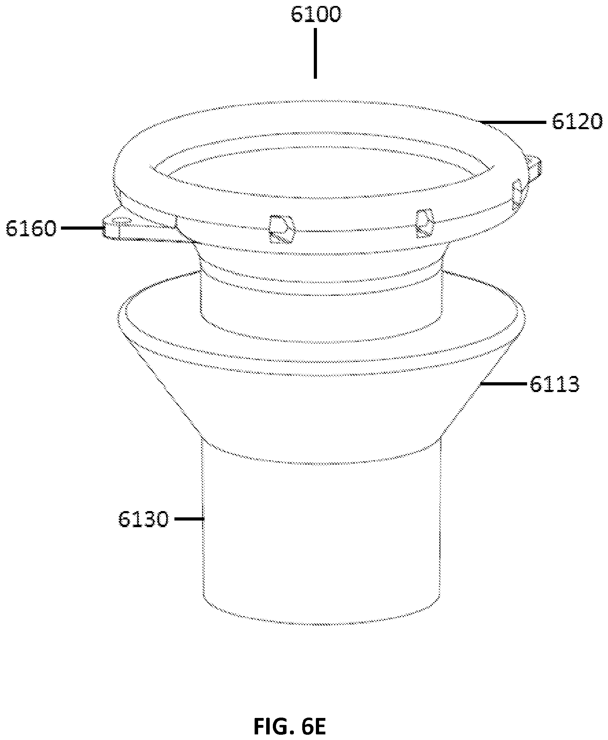

With reference to FIG. **6E****,** a perspective view of a retractor **6100** for use in the natural orifice access device system having still another flange embodiment is shown. In this embodiment, the retractor **6100** is shown with a bolster cone **6113.** The soft thermoset polymer or thermoplastic elastomer bolster cone (tapered with maximum diameter approximately 5.08 cm (2 inches) to approximately 10.16 cm (4 inches) for use in the vagina) may be added to the retractor. In use, the retractor is advanced into the body orifice, such as a vagina, while the bolster cone serves as a stop to externally occlude the vagina. The bolster cone may be fixed or mobile along the tubular body **6130** of the retractor **6100.** The suture ties **6160** prevent the retractor from dislodging and maintain compression of the bolster cone against the orifice opening. The design may optionally include a flange similar to others described herein.

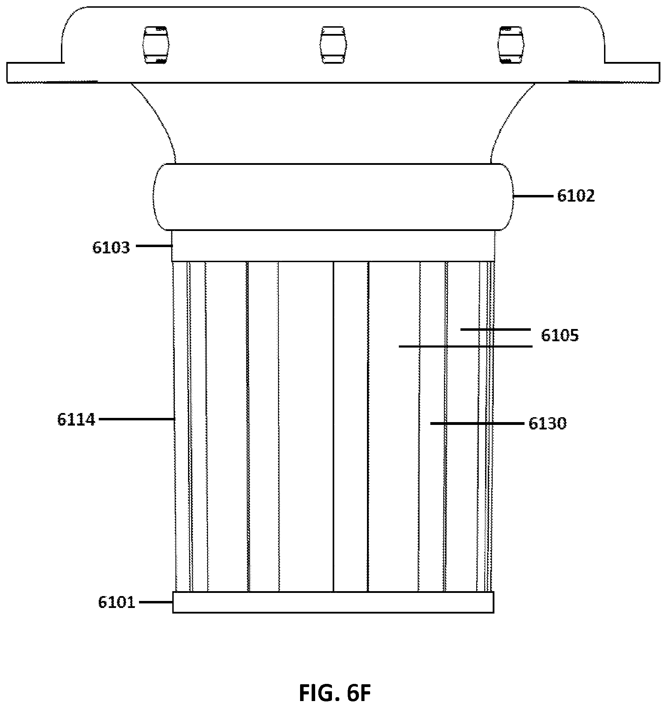

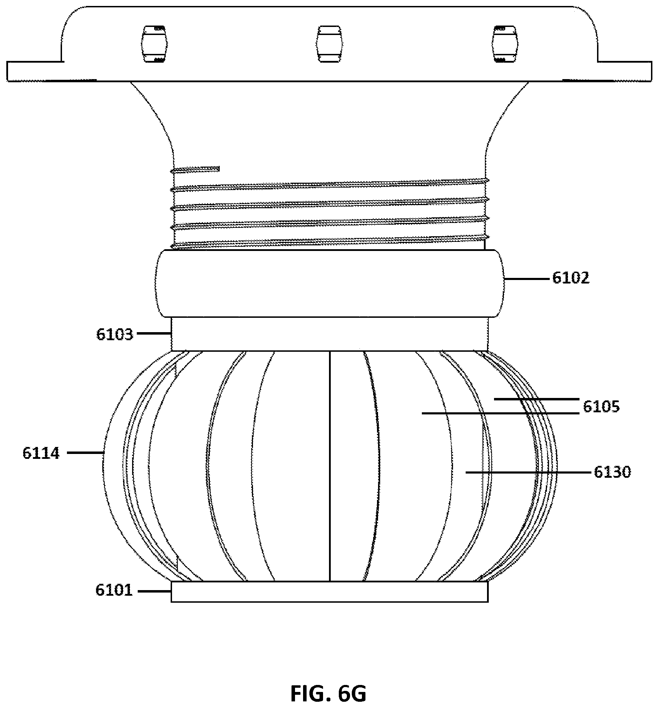

With reference to FIGs. **6F** and **6G****,** a side view of a retractor for use in the natural orifice access device system having a different flange embodiment is shown. In this embodiment, the retractor is shown with a mechanical expanding feature (a "mechanical balloon") **6114.** The mechanical balloon comprises a series of arms **6105** that run circumferentially around the channel **6130,** the arms attached at to a first ring **6103** and a second ring **6101.** The first ring is movable along the length of the channel, while the second ring is fixed at or near the distal end of the channel. A sliding third ring **6102,** disposed around the tubular channel proximal to the first ring, may be advanced toward the distal end of the channel, putting the arms under a load that causes them to flare outwards to assume a balloon-like shape, shown in FIG. **6G****.** The arms exert a force on the anatomy of the natural orifice, causing the retractor to be anchored in place without damaging the surrounding tissue.

In some embodiments, the sliding third ring is threaded along its interior surface and is adapted to engage with threads disposed around the channel. See FIG. **6G****.** In other embodiments, a ratcheting mechanism may be used to move the sliding third ring along the channel.

The sliding ring may comprise any rigid or semi-rigid material, plastic or metal, while the arms may comprise a semi-rigid metal or plastic sufficiently flexible to flare outwards under a load. Alternatively, the arms may comprise a rigid material with an articulating joint located approximately mid-length of each arm, facilitating a similar flaring.

The mechanical balloon has a diameter of approximately 5.08 cm (2 inches) to approximately 10.16 cm (4 inches) and is disposed around the tubular body at the distal end of the retractor. The mechanical balloon may be coated in a nonporous, flexible material to permit occlusion and prevent dislodging when expanded or non-coated to serve solely for fixation. The amount of expansion may be variable to accommodate different anatomies. The feature is initially unexpanded to ease insertion and is then expanded as needed.

In some embodiments, the mechanical balloon may be used in combination with a compression flange as shown in FIG. 6C. In this embodiment, the compression flange is disposed around the tubular body proximal to the mechanical balloon and helps seal the retractor within the natural orifice. In an alternative embodiment, the mechanical balloon may be used in combination with a bolster cone, as shown in FIG. 6E. The bolster cone will act to seal the retractor within the orifice while the mechanical balloon helps anchor it.

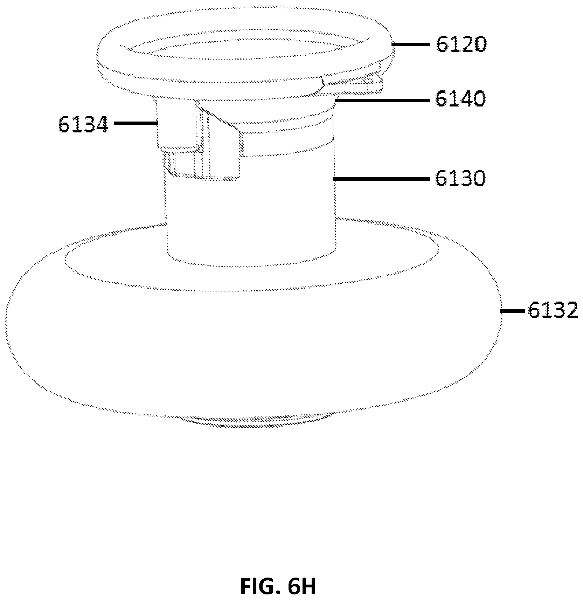

In other embodiments, shown in FIGs. **6H-J,** the flange can comprise an inflatable member **6132** such as an annular balloon coupled to a gas or fluid source that can be selectively inflated and deflated between a deflated, relatively small diameter state for insertion and removal, and an inflated, relatively high diameter state for retention in a body cavity. An inflation port **6134,** for example a check valve, affixed to the funnel portion **6140** of the retractor, is connected to the inflatable member **6132** through a channel **6136** within the wall of the tubular body **6130.** Fluid or gas introduced through the inflation port flows through the channel into the inflatable member to thereby inflate the member.

The channel **6136** runs through the tubular body, generally parallel to the longitudinal axis of the tubular body, with a proximal opening interacting with the inflation port **6134** and a distal opening **6139** into outer surface of the tubular body at the inflatable member. In one aspect, the inflation port **6134** may include a normally closed check valve having a spring-loaded plunger. In a further aspect, the check valve may include a Luer lock. It is contemplated that other inflation ports that are well known in the art may be used.

In this embodiment, the tubular body **6130** is preferably comprised of a relatively rigid material, such as a polycarbonate. The tubular body has an inflatable member at the distal end that may be created by heat shrinking polyolefin tubing around the outside of the tubular body. The distal end of the body/tubing assembly is then heated for approximately 30 to 40 seconds, and then placed inside a mold and injected with air to give the inflatable member an annular balloon shape as seen in FIG. **6H****,** or any other desired shape, depending on the configuration of the mold. The inflatable member **6132** should have sufficient impermeability properties to substantially prevent inflation gas or fluid from permeating through a wall of the inflatable member.

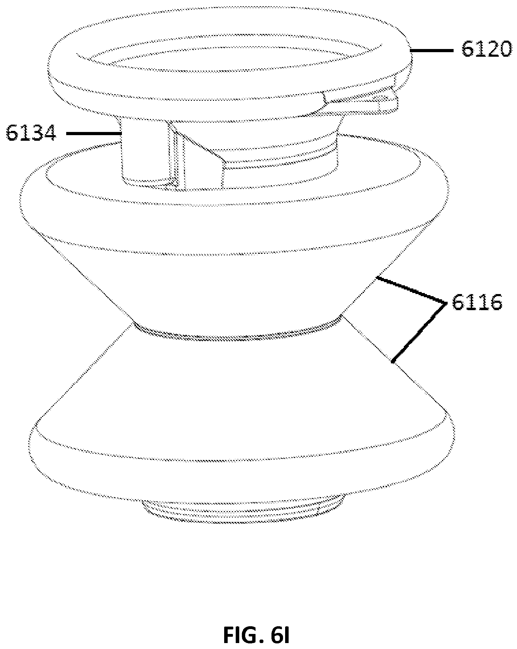

In one embodiment, the inflatable member **6132** may include a substantially toroid shape upon inflation (see FIG. **6H****).** In another embodiment, the inflatable member may include a disc shape upon inflation. In another embodiment, the inflatable member **6132** may be a fluted balloon. In still another embodiment, the inflatable member is a saddle shaped balloon **6116,** as shown in FIG. **6I.** For use in vaginal surgery, the saddle shaped balloon in this embodiment is tapered with maximum diameter from approximately 5.08 cm (2 inches) to approximately 10.16 cm (4 inches). The balloon is initially deflated for ease of entry into the vagina and then may be inflated by through an inflation port 6134 on the proximal end of the retractor. The vaginal opening seats in the low point of the saddle and is compressed between the peaks of the saddle as the balloon is inflated. This occludes the vagina, both internally and externally, and prevents dislodging. The balloon also distributes forces applied during the procedure over a greater area, reducing possible damage to tissue. Suture ties may also be used to mitigate dislodging. The balloon is then deflated upon completion of the procedure to ease removal. Other shapes suitable for particular natural orifices will be appreciated by one skilled in the art.

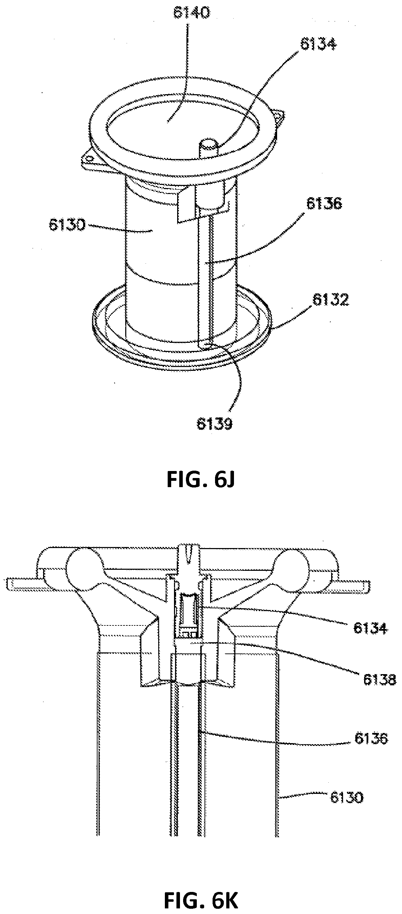

In use, the inflatable member may be inflated after the retractor is disposed within the natural orifice by inserting a syringe into the valve **6134** located at the proximal end **6138** of the channel within the tubular body (see FIG. **6K****).** As shown in FIGs. **6J** and **6K****,** the port leads into the channel **6136,** which allows the fluid or gas from the syringe to travel to the inflatable member **6132.** Alternatively, a bulb pump or other source of gas or fluid may be used to inflate the inflatable member.

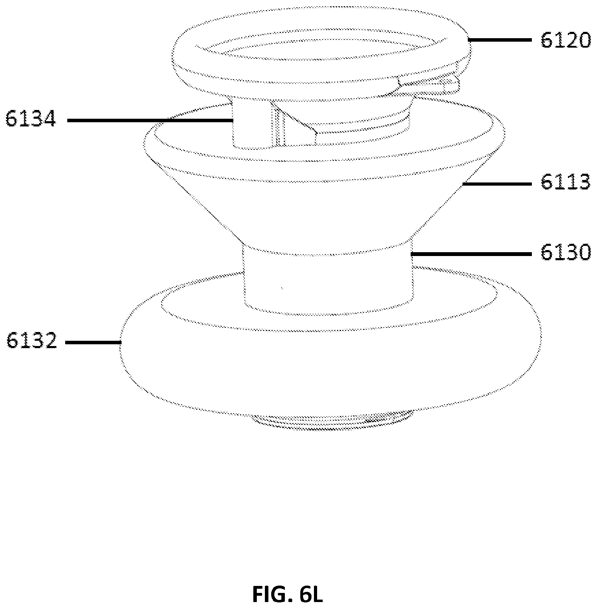

In still another embodiment, the bolster cone described herein may be combined with an inflatable member, as shown in FIG. **6L****.** In this embodiment, the retractor has a balloon **6132** at the distal end and a bolster cone **6113** mid-channel. For use in vaginal procedures, the balloon has a diameter from about 5.08 cm (2 inches) to about 10.16 cm (4 inches) while the bolster cone is tapered with a maximum diameter of about 5.08 cm (2 inches) to about 10.16 cm (4 inches). The balloon **6132** is initially deflated for ease of entry into the vagina and then may be inflated through a port **6134** on the proximal end of the retractor. The amount of inflation may be variable to accommodate different anatomies. The balloon occludes the vagina when inflated and prevents the retractor from dislodging while causing compression of the bolster cone against the vaginal opening to further occlude. The bolster cone distributes forces applied during the procedure over a greater area, reducing possible damage to tissue. Suture ties may also be used to mitigate dislodging. The balloon is then deflated upon completion of the procedure to ease removal.

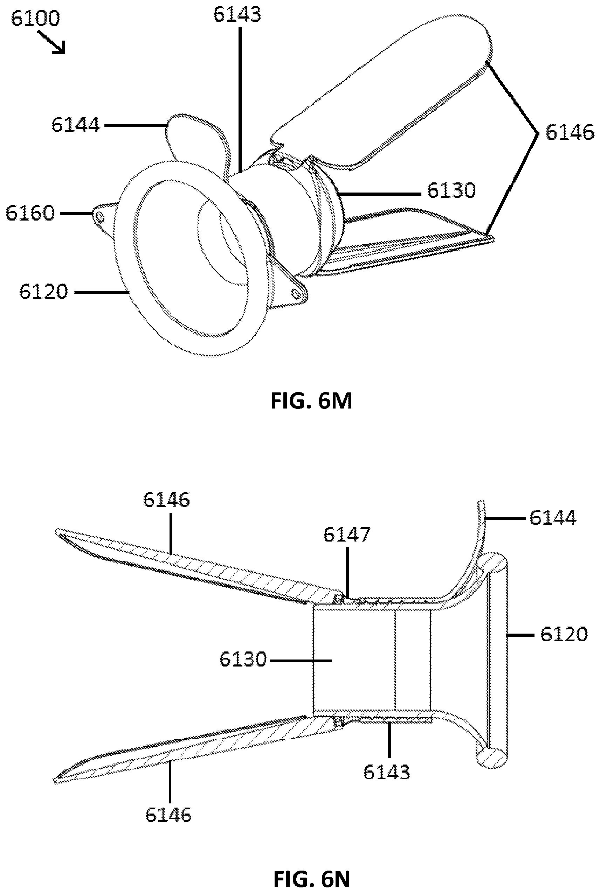

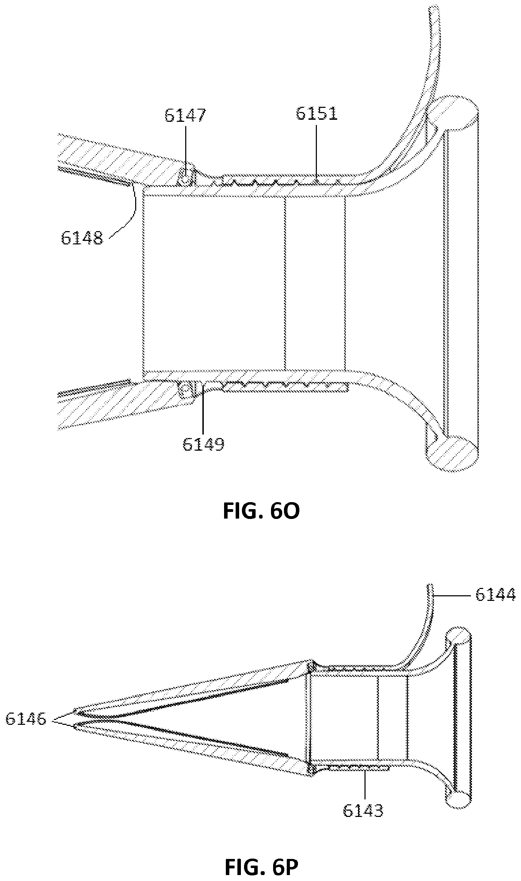

Another embodiment of the natural orifice access system, configured to provide retraction and access without using insufflation gases, is shown in FIGs. **6M-****6P.** In some natural orifices, for example, the vagina, pressure from a standard insufflator can be insufficient to maintain a visible, stable surgical working space due to leaks and billowing created by surges from the insufflator. In this embodiment, the retractor **6100** has a proximal ring **6120** and a tubular body **6130,** with the tubular body adapted to engage a retaining sleeve **6143.** The retaining sleeve includes a lip **6144** at the proximal end and at least two retracting arms **6146** at the distal end. The retracting arms are connected to the retaining sleeve with an articulating hinge **6147** that allows the arms to move between a closed position (FIG. **6P****),** in which the distal ends of the arms in proximity, and an open position (FIGs. **6M****, 6N),** in which the distal ends of the arms are moved away from each other. Optionally, the retracting arms may include an angled lead **6148** near the hinge **6147.**

In the embodiment of FIGs. **6M-6P****,** the tubular body includes an external thread **6149** wrapped around the exterior of the tubular body, adapted to engage an internal thread **6151** disposed around the surface of the interior lumen of the retaining sleeve **6143.** See FIG. **6O****.**

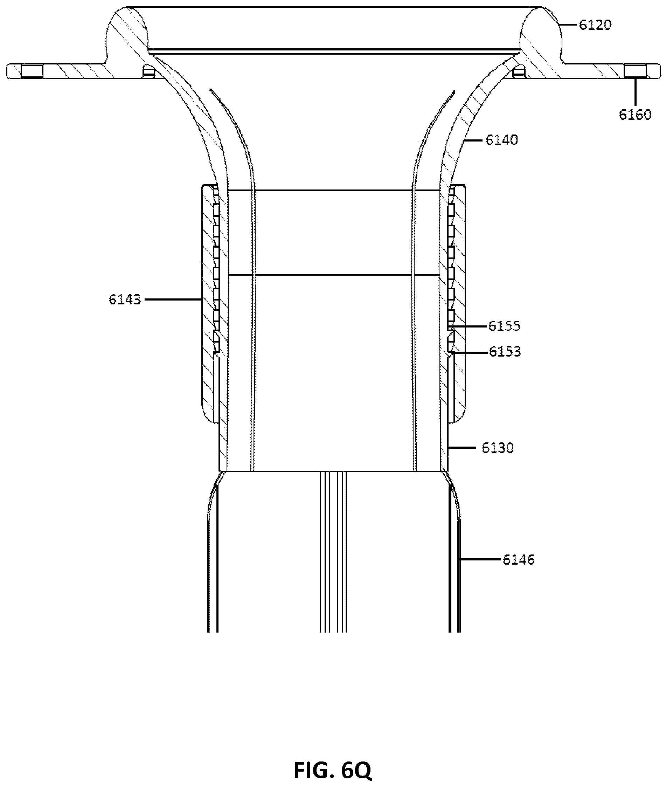

In an alternative embodiment, a ratcheting mechanism comprising teeth **6153** and a series of pawls **6155** may be used instead of internal and external threads (see FIG. **6Q****).** The ratchet mechanism allows the retractor to advance through the lumen of the retaining sleeve with relative ease while resisting backwards movement until the sides of the tubular body of the retractor are manually depressed to release the ratchet mechanism.

In use, the tubular body **6130** of the retractor **6100** is advanced forward through the lumen of the retaining sleeve **6143** until contact is made with the retracting arms **6146.** The angled lead **6148** on the retracting arm allows for the opening to occur at an incremental rate to accommodate a variety of anatomies. The lip **6144** on the retaining sleeve ensures that the proximal end of the lumen will not enter the orifice and provides a holding point during the advancement of the retractor. As seen in FIGs. **6M** and **6N****,** the fully advanced channel presses on the retracting arms, causing them to flare out and hold open the vaginal canal. With the tissue of the vaginal canal retracted, direct access is available to the cervix and surrounding tissue. In FIG. **6P****,** the retractor is drawn back; the retracting arms are disengaged and are able to fold together. This state facilitates insertion and removal from the orifice.

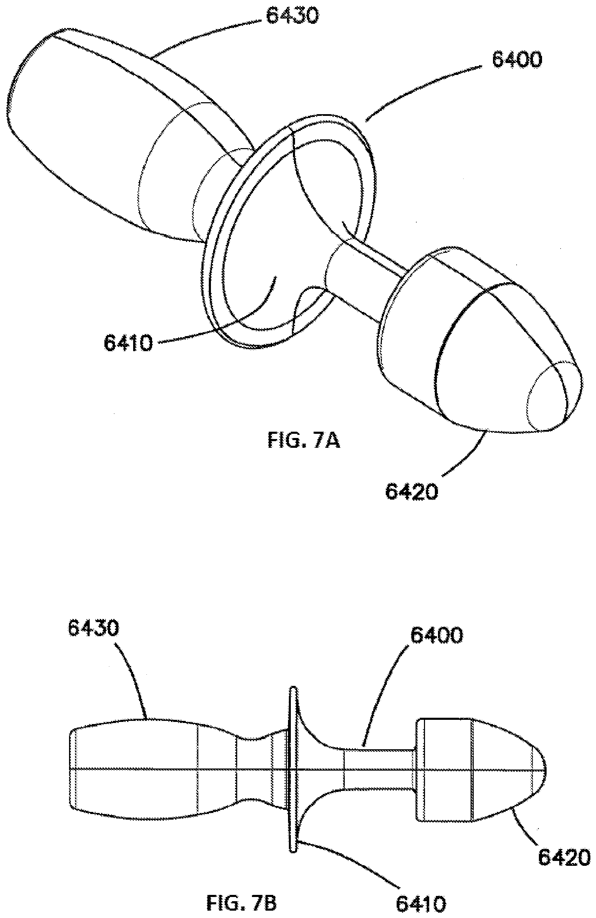

In some embodiments, a natural orifice access system can include a retractor **6100** and an optional obturator **6400** (FIG. **7A-7B****).** The obturator can have a proximal bearing surface **6410** sized and configured to bear against the inner surface **6142** of the funnel segment **6140** and a distal dilation surface **6420** sized and configured to expand a natural orifice for passage of the retractor **6100.** Thus, during insertion of the retractor **6100** into a natural orifice, the dilation surface **6420** expands a pathway to a surgical site in a body cavity while the obturator bears on the inner surface **6142** of the funnel segment **6140** to advance the retractor **6100** into position in the surgical site. Furthermore, in some embodiments, the obturator can have a handle **6430** at a proximal end thereof adapted to facilitate selective twisting or rotation of the obturator about a longitudinal axis thereof during insertion.

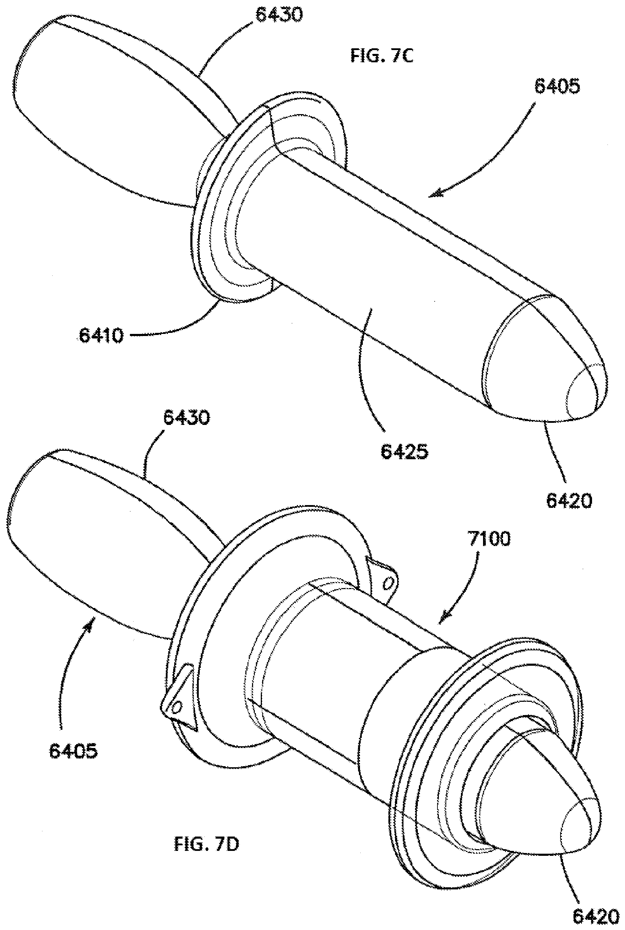

In an alternative embodiment, shown in FIG. **7C****,** the obturator **6405** includes a straight shaft piece **6425** between the distal dilation surface **6420** and the proximal bearing surface **6410** that facilitates dilation of the natural orifice prior to inserting the retractor. It can then be combined with the retractor **6100** to help ease insertion, as shown in FIG. **7D****.**

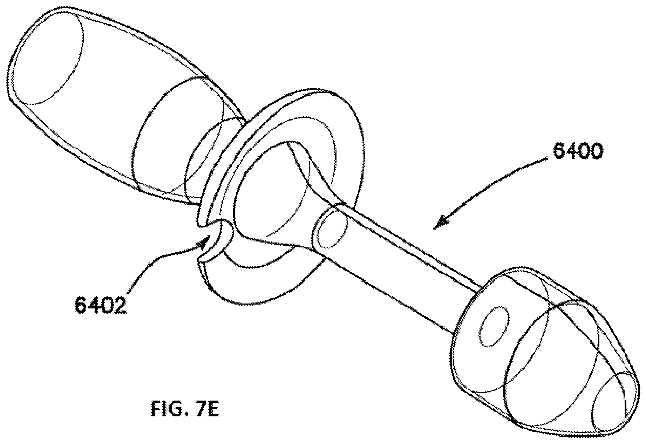

In embodiments having an inflatable member on the retractor, the optional obturator **6400** may be modified with an indent **6139** to provide clearance for the inflation port, as shown in FIG. **7E****.**

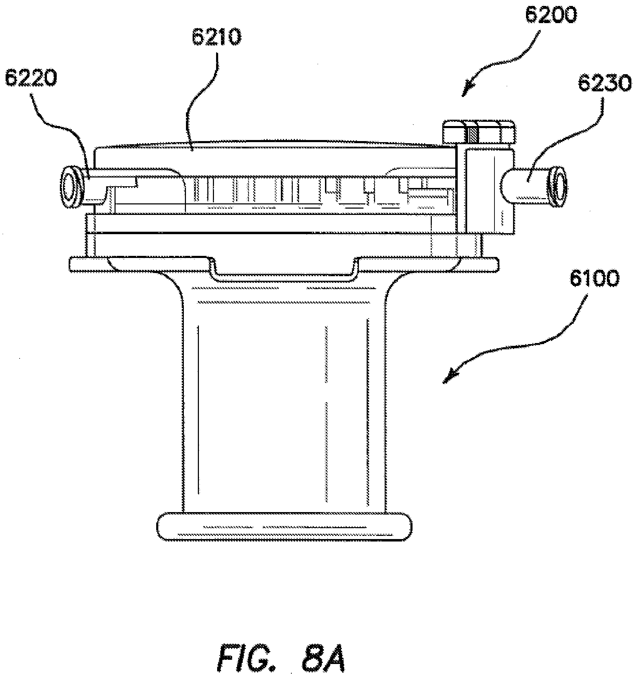

With reference to FIG. **8A****,** a side view of a natural orifice access device having a cap **6200** removably coupled to a retractor **6100** is illustrated. In the illustrated embodiment, the cap **6200** comprises a sealable access surface **6210** such as a gel pad surface as described in further detail herein. In certain embodiments, the cap **6200** can also comprise at least one gas or fluid port **6220, 6230.** In the illustrated embodiment, the cap **6200** comprises two gas or fluid ports **6220, 6230,** such that one port can be used for gas insufflation and the other port can be used for ventilation for example when electrosurgery is performed through the access device. In certain embodiments, at least one of the gas or fluid ports **6220, 6230** comprises a valve such as a stopcock valve to selectively control the flow of fluid there through.

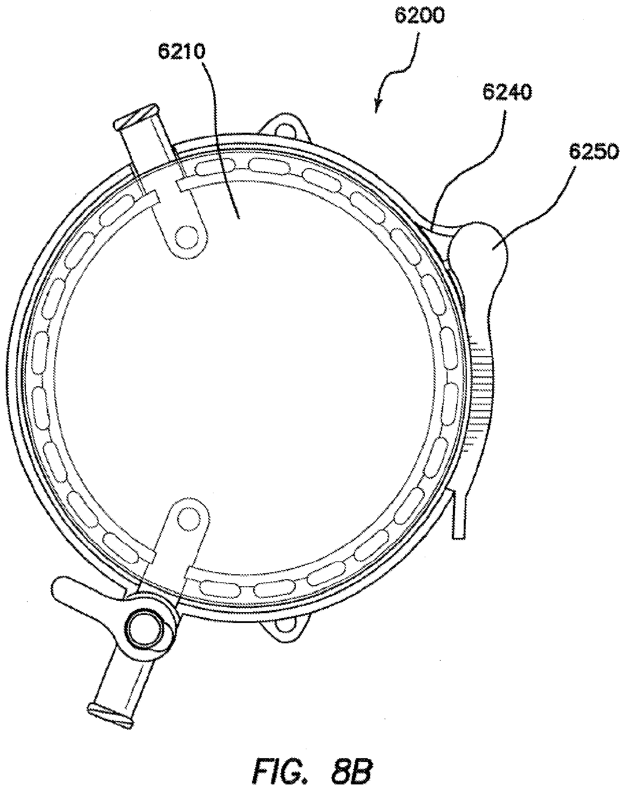

With reference to FIG. **8B****,** a top view of the natural orifice access device is illustrated. The sealable access surface **6210** can be encircled by and restrained by an annular frame **6240** such as a split ring having a clamp **6250.** The clamp **6250** can be movable between an open configuration in which the cap **6200** is selectively removable from the retractor **6100** and a clamped configuration in which the cap **6200** can be secured to the retractor **6100.** For example, the annular frame **6240** can be positioned peripherally around the outer ring **6120** with the clamp **6250** in the open configuration and the clamp moved to the clamped configuration to sealingly fix the cap **6200** to the retractor **6100.** Accordingly, the cap **6200** can be easily removed during a surgical procedure to facilitate removal of excised tissue from a surgical site through the retractor **6100.**

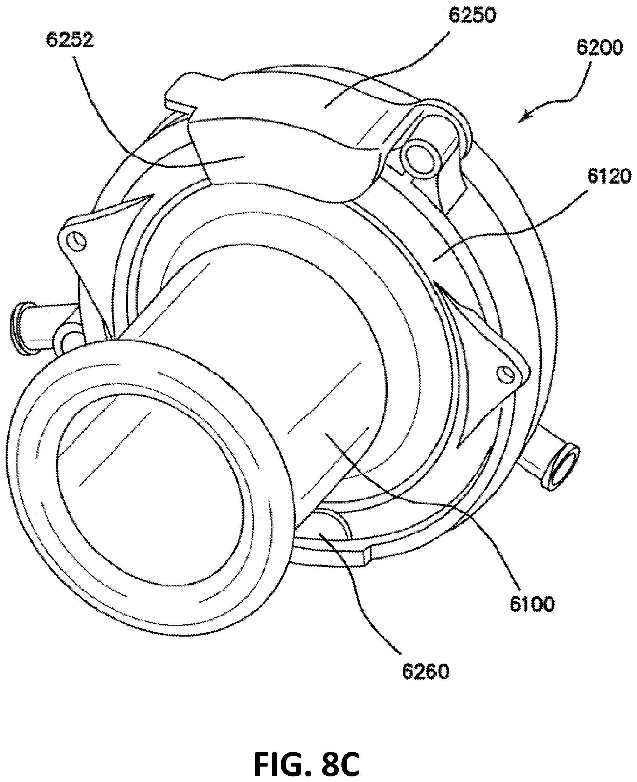

With reference to FIG. **8C****,** a perspective view of the natural orifice access device is illustrated. In the illustrated embodiment, the clamp **6250** can have a distal flange **6252** positioned to interface with the outer ring **6120** of the retractor when the clamp is in the clamped configuration. As illustrated, the clamp **6250** engages a distal surface of the outer ring **6120** of the retractor **6100.** In some embodiments, the annular frame **6240** can further comprise at least one distal flange sized and positioned to interface with a retractor. In the illustrated embodiment, the annular frame **6240** comprises a distal flange **6260** positioned to engage a distal surface of the outer ring **6120** of the retractor. As illustrated, the flange **6260** is generally diametrically opposed to the distal flange of the clamp **6250.** In other embodiments, the annular frame **6240** can include more than one distal flange positioned substantially equally spaced about the periphery of the annular frame **6240** or spaced irregularly about the periphery of the annular frame.

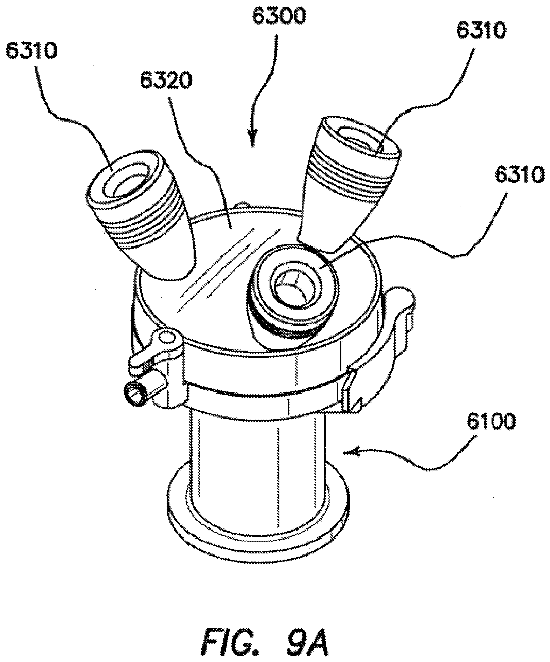

With reference to FIG. **9A****,** another embodiment of natural orifice access device is illustrated with a cap **6300** removably coupled to a retractor **6100.** In the illustrated embodiment, the cap **6300** includes multiple ports **6310** positioned through an access surface **6320** thereof. Advantageously, the multiple ports **6310** allow for easy placement and manipulation of multiple laparoscopic instruments in a surgical site through a single natural orifice.

In the illustrated embodiments of FIG. **9A****,** the ports **6310** have a relatively low profile, that is, protrude minimally above the access surface **6320** and/or below the distal surface of the cap **6300.** Accordingly, the ports **6310** are shorter than a length of a typical trocar and comprise a seal assembly positioned above the access surface **6320** and a cannula extending through the gel pad of the cap **6300.** The reduced length of the ports **6310** allows increased angular or pivotal motion for instruments extending there through, and also permits the use of curved and/or angled instruments.

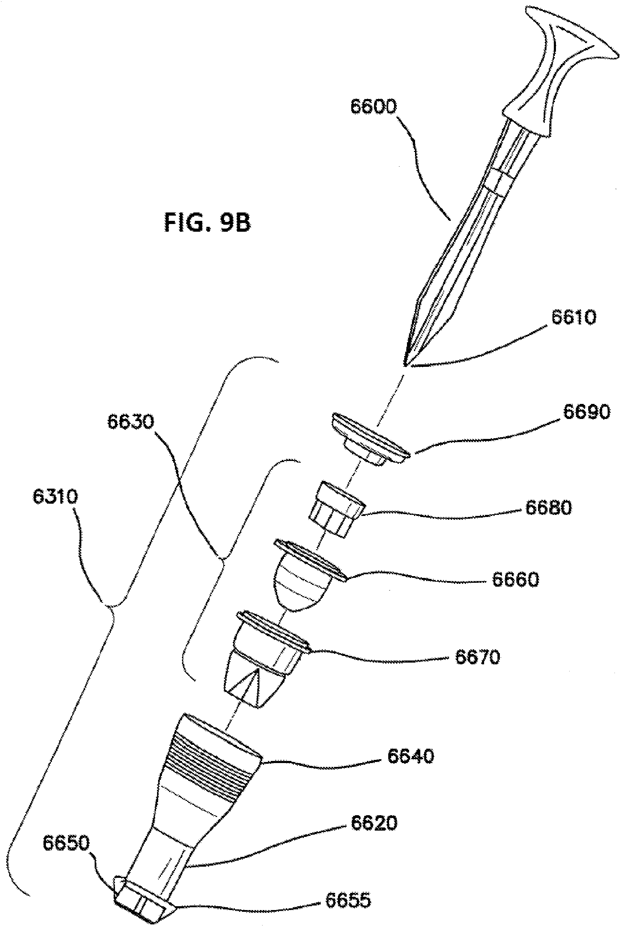

FIG. **9B** is an exploded view of an embodiment of a port **6310** and optional obturator **6600,** which is a component of some embodiments of the access device system. In the illustrated embodiment, the obturator **6600** comprises a pointed, puncture tip **6610.**

The port **6310** comprises a proximal end, a distal end, and a longitudinal axis. The port **6310** comprises a cannula **6620** extending along the longitudinal axis. A seal **6630** is disposed at the proximal end of the cannula **6620,** contained within a housing **6640.** A retainer **6650** is disposed at the distal end or tip of the cannula **6620.**

The cannula **6620** comprises a tubular body dimensioned to accommodate an instrument or instruments received there through. In the illustrated embodiment, the cannula **6620** is a substantially cylindrical tube, and extends through the cap **6300** in use. In the illustrated embodiment, the cannula **6620** is comparatively short because the cannula need only traverse the cap 6300 (FIG. 9A), which has a known and consistent thickness, rather than a body wall. Accordingly, some embodiments of the cannula **6620** are not more than about 2-times longer, about 1.5-times longer, about 1.2-times longer, or about 1.1-times longer than the thickness of the gel pad. In some embodiments, the cannula **6620** is less than about 20 mm, about 10 mm, or about 5 mm longer than the thickness of the gel pad. In some embodiments, the cannula **6620** is about as long as the gel pad is thick. In other embodiments, the cannula **6620** has a different length, for example, a length typical for a cannula used for traversing a body wall. Shorter length cannula permit increased angular degrees of freedom for instruments passing there through. Embodiments of shorter cannula also accommodate curved instruments. The cannula **6620** comprises any suitable biocompatible material. In some embodiments, the cannula **6620** comprises a flexible material.

The illustrated seal **6630** comprises an instrument or septum seal **6660** and a zero seal **6670.** Optionally, a shield **6680** may be disposed within the instrument seal **6660.** The instrument seal **6660** seals instruments passing there through, thereby maintaining pressurization in a body cavity such as pneumoperitoneum or pneumorectum. The zero seal **6670** provides a seal when no instrument passes through the seal **6630.** The instrument seal **6660** and zero seal **6670** are received in a housing **6640** disposed at the proximal end of the cannula **6620** and secured therein by a seal cover **6690.**

The retainer **6650** is disposed at or near the distal end of the cannula **6620.** In some embodiments, the retainer **6650** and cannula **6630** are integrated, while in other embodiments, the retainer **6650** and cannula **6630** are not integrated. In the illustrated embodiment, the proximal end of the retainer **6650** comprises a flange **6655** that is generally flat and perpendicular to the longitudinal axis, while the distal end is tapered, narrowing toward the distal end of the cannula **6620.** The flange **6655** reduces the likelihood of accidental or inadvertent removal of the port **6310** from the cap. Some embodiments of the proximal face of the flange **6655** comprise additional anchoring features, for example, at least one of barbs, spikes, ridges, texturing, and the like, which are configured to penetrate or bite into a distal face of the cap **6300.** In some embodiments, a diameter of the flange **6655** is from about 1.2 to about 2.5 times wider, or from about 1.5 to about 2.0 times wider than an outer diameter of the cannula **6630.** Some embodiments of the port **6310** are 5-mm trocars, in which the outer diameter of the cannula **6620** is from about 7 mm to about 8 mm.

The tapered end of the retainer **6650** facilitates insertion of the port **6310** through the cap, either by itself, or when assembled with the obturator **6600** extending there through. For example, in some embodiments, the retainer **6650** is inserted through a preformed opening in the cap **6300.**

In some embodiments in which the retainer **6650** and cannula **6620** are not integrated, that is, are separate components, the retainer **6650** is secured to the cannula **6620** after the cannula **6620** is inserted through the cap. In some embodiments, the cannula **6620** and retainer **6650** are secured mechanically, for example, using latches, screw threads, clips, lock rings, ratchets, and the like. In some embodiments, the cannula **6620** and retainer **6650** are secured adhesively. In some embodiments, the position of the retainer **6650** is adjustable, for example, to accommodate caps of different thicknesses. In some embodiments, the cannula **6620** and/or retainer **6650** is secured to the cap, for example, adhesively.

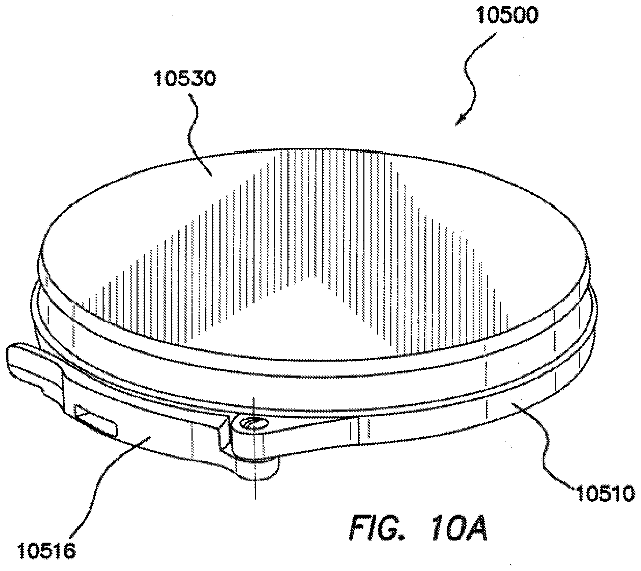

FIG. **10A** illustrates in perspective an embodiment of a cap or cover **10500,** which is a surgical access device that seals the opening between the body cavity and the area outside the body cavity while providing access into the body cavity from outside the body cavity. More particularly, the illustrated cap **10500** releasably and sealingly couples to the outer ring **6120** (FIG. 5), of the wound retractor. The cap **10500** comprises a cap ring **10510** dimensioned and configured for coupling to the outer ring **6120** of the wound retractor and a pad **10530** coupled to the cap ring **10510.** Embodiments of the cap **10500** provide an artificial body wall with consistent properties compared with a natural body wall, for example, thickness, compliance, rigidity, uniformity, and the like.

The illustrated cap or cover **10500** is substantially circular. In other embodiment, the gel cap **10500** has another shape or footprint, for example, oval, elliptical, parabolic, square, rectangular, or another suitable curved or polygonal shape. In some embodiments, the outer ring **6120** of the retractor and cap ring **10510** of the cap have the same general shape or footprint. In other embodiments, the outer ring **6120** of the retractor and cap ring **10501** of the cap have substantially different shapes, for example, a generally circular outer ring **6120** and an oval cap ring **10510.** In these embodiments, the outer ring **6120** is distorted or reshaped for coupling to the cap ring **10510,** for example, by compressing opposed sides of the outer ring **6120.** Non-circular shapes are useful, for example, for procedures in which space is limited. As discussed above, retracting a long, straight incision using an oval or elongated retractor requires less force than a similar procedure using a circular retractor.

In some embodiments, the pad **10530** comprises a gel. In such embodiments, the pad **10530** is referred to as a "gel pad" and the cap **10500** is referred to as a "gel cap". Descriptions of gel pads and gel caps generally apply to embodiments in which the pad **10530** does not comprise gel unless otherwise specified. In some embodiments, the gel pad **10530** does not comprise any preformed access channels there through, for example, for instrument access. Instruments may be inserted directly through the gel pad **10530,** puncturing the gel pad **10530,** and thereby creating access channels or portions in the gel pad **10530.** Each access portion forms an instrument seal in the presence of an instrument inserted there through and a zero seal in the absence of an instrument inserted there through. The gel provides a gas tight seal around a variety of shapes and sizes of instruments inserted there through. Some embodiments of the gel pad **10530** also provide trocar access directly there through, which also provide instrument access into the body cavity. Embodiments of the gel pad **10530** have a working diameter of from about 40 mm to about 120 mm, which is the diameter of a portion of the gel pad **10530** through which instruments and/or trocars may be inserted. Embodiments of the gel cap **10500** are typically from about 10 mm to 50 mm wider than the working diameter.

Accordingly, embodiments of the gel cap **10500** maintain pressurization within a body cavity such as pneumoperitoneum or pneumorectum during multiple instrument exchanges and substantially prevent unintentional loss of pressurization. Embodiments of the gel cap **10500** also provide substantially continuous access and visibility during surgery. Embodiments of the gel cap **10500** have a small profile for use in procedures with limited surgical space.

In some embodiments, the gel is an ultragel, which is characterized by an ultimate elongation greater than about 1000 percent and a durometer less than about 5 Shore A. Some embodiments of the ultragel comprising KRATON^{®} and mineral oil exhibit an ultimate elongation exceeding about 1500 percent and improved sealing properties, for example, sealing with instruments of a wider size range than other seal materials. In some embodiments, the seals comprising ultragels also form zero seals when the instrument is removed therefrom. Accordingly, in some embodiments of seals comprising ultragels, a single seal is acts as both the instrument seal as well as the zero seal.

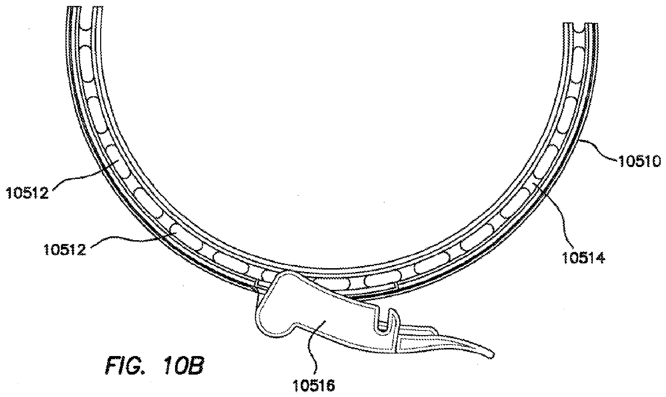

Some embodiments of the cap ring **10510** comprise a substantially cylindrical ring comprising a proximal portion, a distal portion, and a longitudinal axis extending from the proximal portion to distal portions. In other embodiments, the cap ring **10510** has another shape or footprint, for example, oval. As best seen in FIG. **10B****,** which is a bottom view of a cap ring **10510,** in the illustrated embodiment, the proximal portion of the cap ring **10510** comprises a plurality of apertures **10512** distributed about the periphery thereof. The apertures **10512** extend through a wall **10514** at the proximal portion of the cap ring. In other embodiments, the apertures **10512** are disposed in at least one member extending either longitudinally inward or longitudinally outward from the wall **10514** of the cap ring. The gel pad **10530** is disposed at the proximal portion of the cap ring **10510** in the illustrated embodiment, with portions of the gel pad **10530** extending through the apertures **10512,** thereby creating an interlocking structure between the cap ring **10510** and the gel pad **10530,** mechanically locking the cap ring **10510** and the gel pad **10530** together.

The distal portion of the cap ring **10510** is substantially cylindrical in the illustrated embodiment, and is dimensioned and configured to receive the outer ring **6120** (FIG. 5) of the wound retractor. The cap ring **10510** comprises a latch mechanism **10516** that removably couples the cap ring **10510** to the outer ring **6120.** Those skilled in the art will understand that other mechanisms are also useful for coupling the cap ring **10510** to the outer ring **6120** of the wound retractor, for example, protruding lips, levers, clips, latches, tongues, grooves, screw threads, bayonet mounts, screws, friction fittings, compression fitting, snap caps, and the like. In the illustrated embodiment, when the outer ring **6120** of the wound retractor is received in the distal portion of the cap ring **10510,** the outer ring **6120** of the wound retractor contacts and embeds within a portion of the gel pad **10530** disposed at the distal portion of the cap ring **10510,** thereby displacing a portion of the gel, and forming a seal between the gel pad **10530,** and the outer ring **6120** and tubular body **6130** of the wound retractor. Thus, the distal portion of the gel pad **10530** is in juxtaposition with the incision or body orifice. In other embodiments, the cap ring **10510** is permanently coupled or fixed to the outer ring **6120.**

The cap ring **10510** in some embodiments comprises a polymer. Examples of suitable polymers include, at least one of polyethylene (PE), low density polyethylene (LDPE), high density polyethylene (HDPE), ultra high molecular weight polyethylene (UHMWPE), polycarbonate, thermoplastic elastomers (DYNAFLEX^{®}, GLS Corp.; KRATON^{®}, Kraton Polymers), polyphenylene oxide (PPO), polystyrene, and the like. The polymer component of the cap ring is fabricated by any suitable method, including injection molding, melt casting, blow molding, and the like.

Some embodiments of a process in which the gel pad **10530** is cast in the cap ring **10510** are include steps performed at temperatures above about 130 °C over several hours, for example, from about three (3) to about four (4) hours. Accordingly, in some of these embodiments, the cap ring **10510** does not deform under these conditions.

Some embodiments of the gel pad **10530** comprise an elastomeric gel. Examples of such gels are described in U.S. Patent Application No. 10/381,220. Embodiments of the gel are prepared by mixing at least one triblock copolymer with a solvent that dissolves the midblocks of the triblock copolymer. The mixture is typically a slurry. The endblocks typically comprise a thermoplastic material, such as styrene, while the midblocks typically comprise a thermoset elastomer such as, ethylene/butylene, isoprene, or butadiene. Examples of the triblock copolymer include styrene-ethylene/butylene-styrene (SEBS), styrene-isoprene-styrene (SIS), and styrene-butadiene-styrene (SBS). In some embodiments, the solvent is an oil, for example, mineral oil. Upon heating a mixture or slurry of the triblock copolymer, the midblocks dissolve in the mineral oil, thereby forming a network of the insoluble endblocks. The resulting network has enhanced elastomeric properties compared with the parent copolymer. In some embodiments, the triblock copolymer used is KRATON^{®} G1651, which has a styrene to rubber ratio of 33/67. Once formed, the gel is substantially permanent and, by the nature of the endblocks, processable as a thermoplastic elastomer henceforward. The mixture or slurry has a minimum temperature at which it becomes a gel, which is referred to as the minimum gelling temperature (MGT). This temperature typically corresponds to the glass transition temperature of the thermoplastic endblock plus a few degrees. For example, the MGT for a mixture of KRATON^{®} G1651 and mineral oil is about 120 °C. When the slurry reaches the MGT and the transformation to a gel state takes place, the gel becomes more transparent, thereby providing a visual endpoint confirming the complete transformation of the slurry to the gel state, whereupon the gel may be cooled. Some embodiments of the gel comprise a diblock copolymer, either instead of or in addition to the triblock copolymer. Embodiments of the diblock copolymer comprise a thermoplastic first endblock, for example, styrene, and a thermoset elastomeric second endblock, for example, ethylene/butylene, isoprene, or butadiene. An example of a suitable diblock copolymer is styrene-ethylene/butylene (SEB).

For a given mass of slurry to form a complete gel, the entire mass of the slurry is heated to or above the MGT and held at or above the MGT for a sufficient time for the end blocks to form a network or matrix of interconnections. The slurry will continue to form a gel at temperatures between the MGT and temperatures at which the components of the slurry/gel begin to decompose and/or oxidize. For example, when the slurry/gel is heated at temperatures above 250 °C, the mineral oil in the slurry/gel will begin to be volatile and oxidize. Oxidizing may cause the gel to turn brown and become oily.

The speed at which a given volume of slurry forms a gel depends on the speed with which the entire mass of slurry reaches the MGT. Also, at temperatures higher than the MGT, the end block networks distribute and form more rapidly, thereby speeding the gel formation.

The various base gel formulas may also be mixed or alloyed with one another to provide gels with a variety of intermediate properties. For example, KRATON^{®} G1701X is a mixture of seventy percent (70%) SEB and thirty percent (30%) SEBS, with an overall styrene to rubber ratio of 28/72. Those skilled in the art will appreciate that an almost unlimited number of combinations, alloys, and styrene to rubber ratios can be formulated, each providing and embodiment exhibiting one or more advantages, for example, low durometer, high elongation, and good tear strength.

Some embodiments of the gel material further comprise a polymer that, with a foaming agent, improves the sealing properties of the gel, for example, silicone, soft urethanes, and even harder plastics. Examples of suitable silicones include those used for electronic encapsulation. Examples of suitable harder plastics include polyvinylchloride (PVC), isoprene, KRATON^{®} neat, and other KRATON^{®}/oil mixtures. In the KRATON^{®}/oil mixture, suitable oils include vegetable oils, petroleum oils, and silicone oils, as well as mineral oil.

Some embodiments of the gel comprise one or more additives that provide one or more desirable properties, for example, at least one of enhanced lubricity, improved appearance, and wound protection. Additives are incorporated directly into the gel and/or applied as a surface treatment. In some embodiments, other compounds are added to the gel to modify its physical properties and/or to assist in subsequent modification of the surface by providing bonding sites and/or surface charges. Additionally, oil-based colorants are added to the slurry to create gels of different colors in some embodiments.

Some embodiments of the gel pad **10530** comprise a layer of polyethylene on at least one surface. Polyethylene is dissolved in mineral oil and the solution applied to one or more surfaces of the gel pad **10530.** The mineral oil does not evaporate, but instead, absorbs into the gel pad over time, leaving behind the polyethylene as a layer on the surface of the gel pad.

In some embodiments, the triblock copolymer/solvent mixture/slurry used to manufacture the gel pad **10530** comprises about ninety percent (90%) by weight of mineral oil and about ten percent (10%) by weight of KRATON^{®} G1651. From a thermodynamic standpoint, this mixture behaves similarly to mineral oil. Because mineral oil has a relatively high heat capacity, transforming 0.45 kg (1 pound) of the slurry into a homogenous gel at about 130 °C may take from bout three (3) to about four (4) hours. Once formed, the gel can be cooled as quickly as practicable with no apparent deleterious effects on the gel. In some embodiments, the gel is cooled by cold-water immersion. In other embodiments, the gel is air-cooled. Those skilled in the art will recognize that other cooling techniques are used in other embodiments.

Certain properties of the KRATON^{®}/oil gel will vary with the weight ratio of the components. In general, a higher proportion of mineral oil results in a softer gel, while a higher proportion of KRATON^{®} results in a firmer gel. A too-soft gel exhibits excessive tenting or doming of the gel cap **10500** during surgery when a patient's body cavity is insufflated. Some embodiments of gels that are too soft also do provide an adequate instrument seal and/or zero seal. The gel should be sufficiently soft to provide an adequate seal both in the presence of an instrument and in the absence of an instrument, however.

On prolonged or extended sitting or standing, the copolymer, such as KRATON^{®}, and the solvent, such as mineral oil, in the slurry may separate. The slurry may be mixed to greater homogeneity, for example, with a high shear mixer. Mixing the slurry may introduce or add air to the slurry, however. To remove air from the slurry, the slurry may be degassed. In some embodiments, the slurry is degassed under a vacuum, for example, within a vacuum chamber. In some embodiments, the applied vacuum is about 0.79 meters (about 29.9 inches) of mercury, or about one (1) atmosphere. Optionally, stirring or mixing the slurry under vacuum facilitates removal of the air. During degassing under vacuum, the slurry typically expands, then bubbles, and then reduces in volume. The vacuum is typically discontinued when the bubbling substantially ceases. Degassing the slurry in a vacuum chamber reduces the volume of the slurry by about ten percent (10%). Degassing the slurry also reduces oxidation of the finished gel in some embodiments.

Degassing the slurry tends to result in a firmer gel. A gel made from a degassed slurry comprising about 91.6% by weight of mineral oil and about 8.4% by weight of KRATON^{®} G1651, an eleven-to-one ratio, has about the same firmness as a gel made from a slurry that is not degassed and that comprises about ninety percent (90%) by weight of mineral oil and about ten percent (10%) by weight of KRATON^{®} G1651, a nine-to-one ratio.

Because mineral oil typically has a lower density than KRATON^{®}, the two components will separate after mixing, with the less dense mineral oil rising to the top of the container. This phase separation typically occurs when transforming a static slurry into a gel over several hours. Consequently, the resulting gel is nonhomogeneous, with a higher concentration of mineral oil at the top and a lower concentration at the bottom. The speed of separation is a function of the depth or head height of the slurry being heated. Factors relevant to the relative homogeneity of the gel include the mass of slurry, the head height, the temperature at which the gel sets, and the speed at which the energy is transferred to the gel.

The gel pad **10530** or gel cap **10500** are gamma sterilized in some embodiments, which is relatively and/or comparatively simpler to qualify compared with other sterilization process, for example, versus ethylene oxide. Gamma sterilization can cause large bubbles to form in the gel pad, however, which are cosmetic and/or aesthetic issues in the sterilized devices. Because bubbles typically comprise greater than ninety-nine percent (99%) room air, the dissolved air is advantageously removed from the slurry prior to transforming the slurry into a gel. For example, the slurry may be degassed under vacuum, as described above, then gelled by heating. Some bubbles may still form in the gel during gamma sterilization, but typically disappear over a period of from about twenty-four (24) hours to about seventy-two (72) hours. Typically, mineral oil at room temperature has about ten percent (10%) dissolved gas. As discussed above, removing air from the gel makes the gel firmer. This effect is counterbalanced by a softening of the gel by the gamma radiation during gamma sterilization, however.

In some embodiments in which the gel pad **10530** is gamma sterilized, the gel comprises about ninety percent (90%) mineral oil by weight and about ten percent (10%) KRATON^{®} by weight. As stated above, degassing the slurry makes the gel firmer. The counteracting softening by the gamma radiation, however, results in a gel with substantially the same firmness as a gel comprising about ninety percent (90%) mineral oil by weight and about ten percent (10%) KRATON^{®} by weight that is not degassed and gamma sterilized.

In some embodiments, the gel pad **10530** is coupled to, attached to, formed with, or integrated with the cap ring **10510** to provide a gas-tight seal between the cap ring **10510** and the tubular body **6130** (FIG. 5). The gel pad **10530** covers and seals the entire opening in the cap ring **10510,** as well as covering substantially the entire wound or orifice opening. As stated above, the gel pad **10530** provides a gas tight seal around a variety of shapes and sizes of instruments inserted there through.

Embodiments in which a gel pad support structure of the cap ring **10510** comprises a thermoplastic elastomer, for example, DYNAFLEX^{®} or KRATON^{®} , and the gel pad **10530** comprises a similar thermoplastic elastomer, for example, KRATON^{®}, exhibit improved adhesion between the gel pad **10530** and the cap ring **10510.** The polystyrene component of KRATON^{®} in the gel pad **10530** improves adhesion with polyphenylene oxide (PPO), polystyrene, and other similar polymers.

In some embodiments of cap rings **10510** comprising polycarbonate, the polycarbonate component of the cap ring **10510** does not bond with the gel pad **10530** at 130 °C, which is a typical manufacturing temperature for a gel pad **10530** comprising KRATON^{®}. Raising the temperature to about 150 °C for a few minutes during casting, however, bonds the gel pad **10530** to the cap ring **10510.** It is believed that heating the gel pad **10530** and cap ring **10510** to a temperature at which both the polystyrene component of the gel and the polycarbonate are simultaneously above their melt points allows bonds to form there between. In other embodiments, the uncured gel and the cap ring **10510** are heated to near or at the glass transition temperature of the polycarbonate in the cap ring **10510,** thereby bonding the gel pad **10530** to the cap ring **10510.**

In some embodiments, the gel comprises mineral oil and the cap ring **10510** comprises a polymer that dissolves in mineral oil under the manufacturing conditions, for example, polyethylene (PE), low density polyethylene (LDPE), high density polyethylene (HDPE), and ultra high molecular weight polyethylene (UHMWPE). Using polyethylene (PE) as an example, PE has a higher molecular weight than mineral oil and dissolves in mineral oil at the temperatures used to cast the gel pad **10530.** As such, as a portion of the PE in the cap ring **10510** dissolves in the mineral oil in the gel pad **10530** at the processing temperatures, for example, above about 130 °C, a bond between the PE in the cap ring **10510** and gel pad **10530** is formed.

In an embodiment of a method for manufacturing a gel cap, the cap ring **10510** is placed into a mold that together with the cap ring **10510** includes a negative space in the desired shape of the gel pad and uncured gel is added to the mold. Sufficient uncured gel is then added to the mold to cover and fill the apertures **10512.** The uncured gel flows through, fills, and remains within the apertures. Also, in some embodiments, the mold is filled with sufficient uncured gel to extend into the distal portion of the cap ring **10510.** After the gel cures, the gel in the apertures connects and couples the gel on a first side of each aperture **10512to** the gel on a second side of the aperture, thereby mechanically locking the gel pad **10530** to the cap ring **10510.**

Some embodiments include another method for coupling the gel pad **10530** to the cap ring **10510,** either in addition to or instead of the mechanical interlocking discussed above. Such methods are useful, for example, for coupling separately formed gel pads or gel slugs **10530** and cap rings **10510.** Some embodiments use a glue or adhesive to couple the gel pad **10530** to the cap ring **10510,** for example, cyanoacrylate (SUPERGLUE^{®} or KRAZY GLUE^{®}). The glue is believed to bond to either the rubber or the styrene component of the triblock copolymer with a bond is frequently stronger than the gel material itself. Some embodiments use solvent welding in which a solvent dissolves a plastic in the cap ring **10510** and the polystyrene in the gel pad **10530.** The solvent is applied to the gel pad **10530** and cap ring **10510** by any suitable method, for example, by spraying and/or by dipping. In effect, the solvent melts both the plastic of the cap ring **10510** as well as the polystyrene in the gel pad **10530,** thereby forming a bond between the two, which remains after the solvent evaporates.

In an embodiment for manufacturing a gel cap **10500,** the gel pad **10530** is cast into the cap ring **10510** to form the gel cap **10500.** The cap ring **10510** is positioned in or placed into a mold cavity of a casting mold. Embodiments of the mold cavity include support for the annular walls of the cap ring **10510.** Embodiments of the mold comprise a material with sufficient heat dissipation properties, for example, at least one of aluminum, copper, and brass. Those skilled in the art will recognize that other mold materials with lower heat dissipation properties will produce acceptable parts in some embodiments. Furthermore, some embodiments of the mold comprise active cooling elements, for examples, channels through which coolants are pumped.

The mold cavity and cap ring **10510** assembly is then filled with a desired amount of the triblock copolymer/mineral oil slurry such that the slurry contacts the cap ring **10510.** In some embodiments, the slurry is preheated, for example, to about 52 °C (125 °F), which facilitates a complete filling of the mold cavity by the slurry, thereby reducing the probability of voids in the gel. Preheating the slurry to a temperature below the MGT reduces the viscosity of the slurry and allows the slurry to flow more easily. As stated above, some embodiments of the slurry are degassed in a vacuum before casting. In some embodiments, the slurry is also degassed after it is filled in the mold cavity to remove any air that may have been introduced during the filling of the mold cavity, as well as to facilitate flow of the slurry into voids in the mold. The mold, cap ring, and slurry are heated, for example, in an oven, until the slurry reaches a temperature of about 150 °C. As stated above, the slurry turns into gel at about 120 °C; however, at about 150 °C, the gel bonds to a polycarbonate cap ring **10510.** Depending on the material used in the cap ring **10510,** bonding may take place at a temperature other than about 150 °C. In embodiments in which the cap ring **10510** is comprises a material with a lower melting point than the MGT, for example 120 °C, the gel pad **10530** is molded separately as a gel slug, which is then bonded to the cap ring **10510** as discussed above.

When the transformation of the slurry into a gel is complete, for example, when the temperature of the gel pad reaches about 150 °C, the gel cap **10500** is cooled, for example, by air-cooling, cold-water immersion, or another suitable method. At 150 °C the gel pad **10530** is soft and easily distorted. Distortions in the gel pad **10530** present during cooling would be set after cooling. Accordingly, in some embodiments, the gel cap **10500** is cooled within the mold, thereby reducing the likelihood of distorting the gel pad **10530.** Factors affecting the cooling time include the size and configuration of the mold, the quantity of gel, temperature and quantity of cooling medium, the properties of the cooling medium, and the mold material. As an example, the cooling time for a particular gel cap **10500** may be about two (2) hours for air cooling and about fifteen (15) minutes for water cooling. Whether cooling with air or water, the final properties of the gel are substantially the same. The gel cap **10500** is typically cooled to about ambient room temperature, but may be cooled to a lower temperature if desired. At about 0 °C, the gel hardens, which is useful, for example, in secondary operations such as when coupling separately manufactured gel pads **10530** and cap rings **10510.** The gel cap **10500** may be removed from the mold at any time after the gel has set.

When removed from the mold, the gel pad **10530** typically has a tacky surface. Coating the gel pad **10530** with a powder, such as cornstarch, substantially reduces or eliminates the tackiness of the cured gel pad **10530.**

As stated above, in some embodiments, the gel pad **10530** is molded separately from the cap ring **10510,** and coupled to the cap ring **10510** in a secondary operation, for example, bonding. In some embodiments, the gel pad **10530** is molded as a gel slug with an outer perimeter smaller than the perimeter of the inner cylindrical wall of the cap ring **10510** and a height greater than the height of the cap ring **10510.** Because the gel pad **10530** is molded separate from the cap ring **10510,** the slurry need only be heated to the MGT, for example, about 120 °C, to complete the transformation of the slurry into a gel, whereupon the gel becomes substantially transparent. As discussed above, the gel slug may be cooled, for example, to about 0 °C, then placed within the inner cylindrical wall of the cap ring **10510.**

In some embodiments, the gel slug is coupled to the cap ring **10510** through compression molding, in which the gel slug is compressed longitudinally, thereby expanding the outer perimeter of the gel slug and compressing the gel slug against the inner cylindrical wall of the cap ring **10510.** The compressed gel slug and cap ring **10510** are then heated to a sufficient temperature for the polystyrene in the gel and the polymer of the cap ring **10510** to form bonds there between. Molding the gel slug separately from the cap ring **10510** followed by heat bonding the gel slug to the cap ring is especially useful in embodiments in which the cap ring **10510** comprises a material with a melting temperature lower than the MGT of the gel. In such situations, the gel slug can be molded separately and heat bonded to the cap ring **10510** without melting the cap ring **10510.**

An embodiment of a method for retracting an incision or body orifice using the retractor **6100, 7100** is discussed in detail above. The method results in the outer ring **6120** of the retractor substantially in contact with the exterior surface of the body wall. The gel cap **10510** is then coupled to the outer ring **6120** of the retractor, thereby sealing the opening between the body cavity and the area outside the body cavity and allowing the surgeon to insufflate the body cavity.

As discussed above, embodiments of the gel cap **10500** comprise no preformed access channels in the gel pad **10530.** In use, instruments may be inserted directly through the gel pad **10530,** thereby creating access channels through the gel pad **10530.** Each access channel created in the gel cap forms an instrument seal in the presence of an instrument passing there through because the gel provides a gas tight seal around a variety of shapes and sizes of instruments. When the instrument is removed from the gel pad **10530,** the channel created in the gel pad by the instrument closes to form a zero seal.

Some embodiments of the cap use access devices such as trocars inserted through the gel pad **10530** for instrument access, in particular, where an access channel experiences repeated instrument manipulation, for example, insertion, removal, advancement, retraction, rotation and/or other manipulation. Each trocar inserted through the gel pad **10530** permits repeated introduction, removal, and/or manipulation of instruments there through.

In some embodiments, the gel cap **10500** initially comprises no access channels, and the surgeon is at liberty to determine the placement of instruments there through. Moreover, the surgeon has unlimited flexibility in the placement and repositioning of ports within the area of the gel cap **10500,** as well as the option of selecting different trocar sizes for different clinical procedures. Being detachable, the gel cap **10500** allows for the removal of large specimens. Once removed, the gel cap **10500** can be re-coupled to the outer ring **6120** of the retractor, thereby restoring the seal and allow the surgeon to re-insufflate the body cavity.

Moreover, embodiments of the gel are deformable without losing physical integrity, and while maintaining substantially gas tight instrument seals with any instruments extending there through, as well as gas tight zero seals for any access channels without any instruments extending there through. Accordingly, embodiments of the gel cap **10500** permit both translational or positional, and angular or pivotal "float" or degrees of freedom for the instruments passing through the gel pad **10530.** This float permits instrument motion both relative to the cap ring **10510** as well as relative to other instruments. In contrast, other single or limited port systems do not exhibit one or both translational or angular float for instruments.

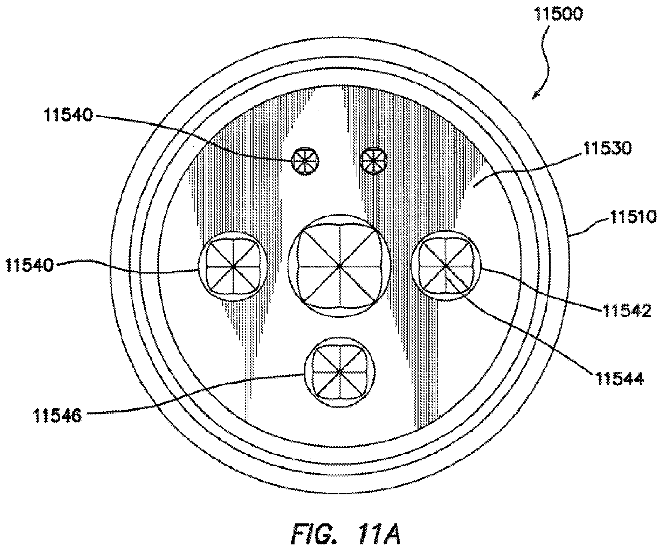

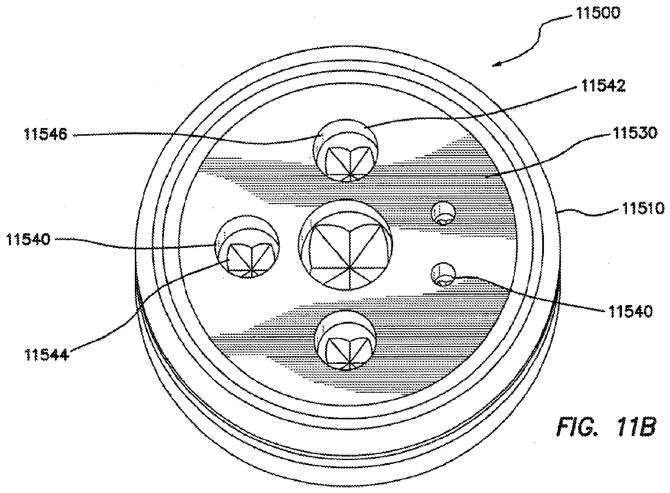

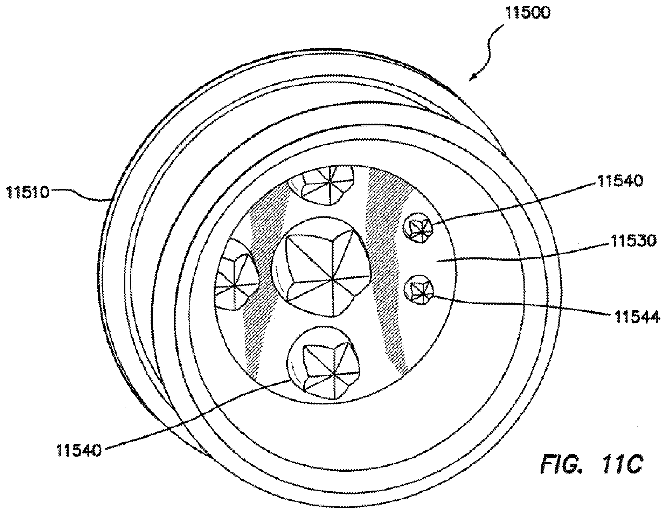

FIG. **11A** is a top view of an embodiment of a gel cap **11500** comprising a plurality of access ports, seals, or sealing valves disposed in the gel pad. FIG. **11B** is a perspective top view of the gel cap **11500** mounted on a retractor. FIG.11C is a perspective bottom view of the gel cap **11500** mounted on a retractor. The gel cap **11500** comprises a cap ring **11510** and a gel pad **11530,** which are generally similar to the cap ring and gel pad of the embodiment described above.

The gel cap **11500** further comprises a plurality of access ports **11540,** at least a portion of which is disposed within or embedded within the gel pad **11530.** In the illustrated embodiment, the access ports **11540** have a low profile, that is, do not protrude or protrude minimally above the proximal surface of the gel pad **11530** and/or below the distal surface of the gel pad **11530.** Accordingly, the lengths of the access ports **11540** are similar to the thickness of the gel pad **11530,** which is shorter than a length of a typical trocar inserted in the gel pad **11530,** which comprises a seal assembly positioned above the gel pad **10530,** and a cannula extending through the gel pad **11530.** The reduced length of the access port **11540** allows increased angular or pivotal motion for instruments extending there through, and also permits the use of curved and/or angled instruments. In the illustrated embodiment, the access ports **11540** are substantially permanent or non-removable under the conditions under which the gel cap **11500** is used. Trocars can also be inserted through the gel pad **11530** if additional ports are desired.

Each port **11540** comprises longitudinal axis extending from a proximal side to a distal side of the gel pad **11530,** a first seal **11542** disposed at the proximal side of the gel pad **11530,** and a second seal **11544** disposed distal to the first seal **11542.** A sight of each of the ports or seals **11540** has an aperture through the gel pad **11530** and coincides with the longitudinal axis. In the illustrated embodiment, the first seal **11542** forms an instrument seal with an instrument extending there through and the second seal **11544** forms a zero seal in the absence of an instrument extending there through.

In the illustrated embodiment, the first seal **11542** comprises a septum seal. Each septum seal comprises an aperture **11546** there through that is slightly smaller than a cross-section of the smallest instrument to be inserted there through. The aperture **11546** of the septum seal is substantially aligned with the aperture through the gel pad and the longitudinal axis of the port **11540.** When an instrument is inserted through the aperture **11546** of the septum seal, the aperture **11546** expands and engages the outer surface of the instrument, thereby forming a seal therewith. The septum seal comprises an elastomeric material that biases the aperture against an instrument is inserted there through. Those skilled in the art will understand that other types of instrument seals are used in other embodiments.

In the illustrated embodiment, the second seal **11544** comprises a double-duckbill valve, which functions as a zero-closure seal that provides a zero seal in the absence of an instrument inserted there through. Those skilled in the art will understand that the second seal comprises another type of seal, for example, a duckbill valve, a flap valve, and the like. The double-duckbill valve comprises as elastomeric material. In some embodiments, each of the first seal **11542** and the second seal **11544** independently comprise an elastomeric material, for example, at least one of rubber, synthetic rubber, silicone, ethylene propylene diene monomer (EPDM), ethylene-propylene copolymer (EP rubber), polyisoprene, polybutadiene, polyurethane, styrene-butadiene, ethylene vinyl acetate (EVA), polychloroprene (NEOPRENE^{®}), perfluorelastomer (KALREZ^{®}), and the like

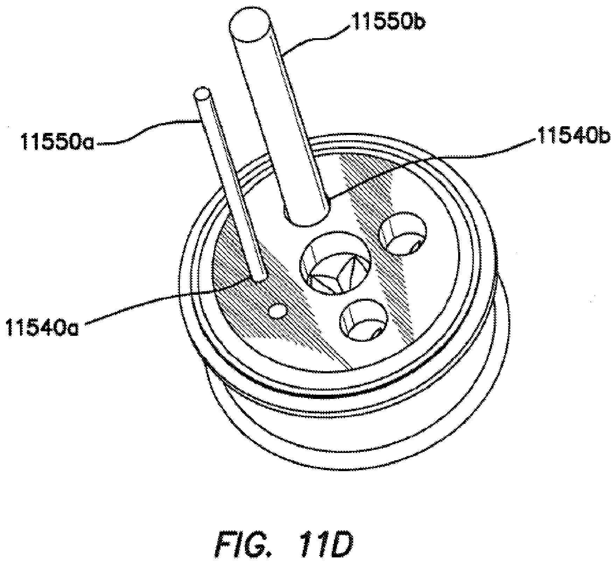

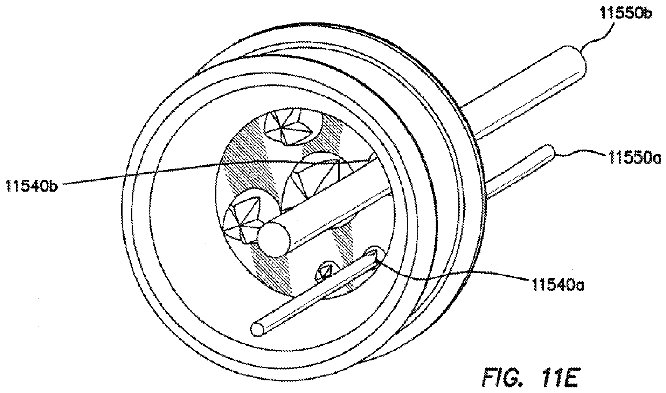

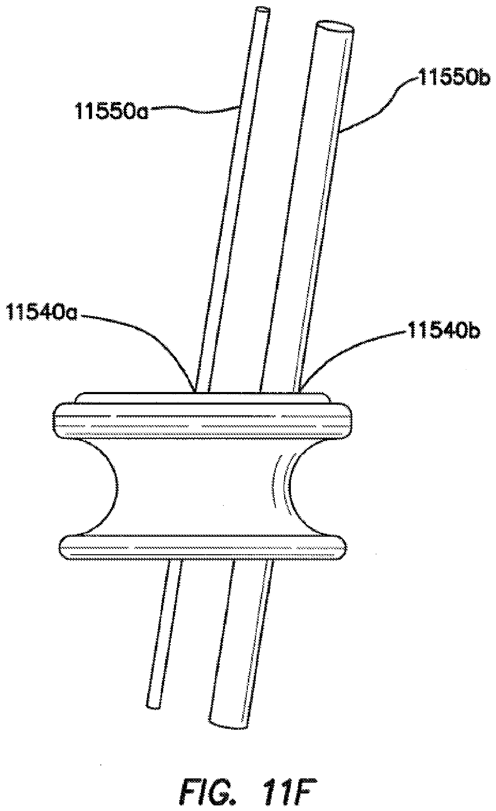

Thus, during use, the septum seal provides an instrument seal in the presence of an instrument inserted there through, and the duckbill valve provides a zero seal in the absence of an instrument inserted there through. The illustrated embodiment comprises ports or seals **11540** in the gel pad of different sizes. Each size of port **11540** sealing accommodates a different range of instrument sizes inserted there through. The size of a port is typically given as the diameter of the largest instrument that the port will accommodate, for example, 5 mm, 11 mm, or 12 mm. FIGS. **11D****,** **11E****,** and **11F** are a perspective top view, a perspective bottom view, and a side view of a thinner instrument **11550a** and a thicker instrument **11550b** inserted through a smaller port **11540a** and a larger port **11540b,** respectively, of the embodiment of the gel cap **11500** illustrated in FIGS. **11A-11C****.**

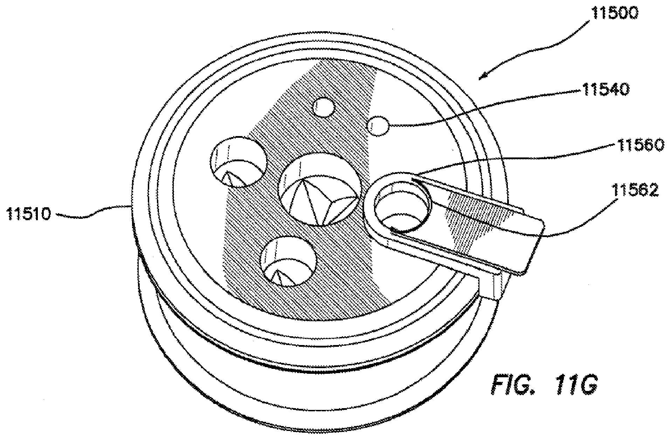

FIG. **11G** is a top perspective view of an embodiment of a gel cap **11500** further comprising a fixed port position, for example, for a camera or a laparoscope. The fixed port **11560** comprises a lock mechanism **11562** that maintaining the position of a camera or laparoscope inserted there through. In some embodiments, one of the ports **11540** further comprises a stopcock and/or gas fitting used as a gas inlet and/or outlet port for insufflating, depressurizing, and/or venting the body cavity of gas. In some embodiments, a gas inlet/outlet port is disposed on the cap ring **11510.**

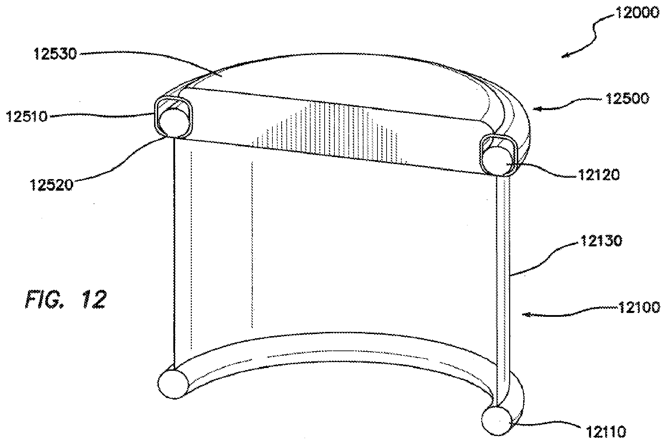

FIG. **12** is a cutaway perspective view of an embodiment of an access device system **12000** comprising retractor **12100** and a cap or cover **12500,** which are similar to embodiments of retractors and gel caps described above. The retractor **12100** comprises an inner ring **12110,** an outer ring **12120,** and a sleeve **12130** extending between the inner ring **12110** and the outer ring **12120.** In the illustrated embodiment, the cap **12500** is a gel cap comprising a proximal side, a distal side, a cap ring **12510,** and a gel pad **12530.** In the illustrated embodiment, the cap ring **12510** comprises a tubular ring dimensioned to receive the outer ring **12120** of the retractor therewithin. The distal side of the cap ring **12510** comprises an annular slot **12520,** which is sufficiently radially deformable for the outer ring **12120** to reversibly pass there through. Accordingly, the illustrated embodiment of the cap ring **12510** secures the cap **12500** to the outer ring **12120** with a snap or friction fit.

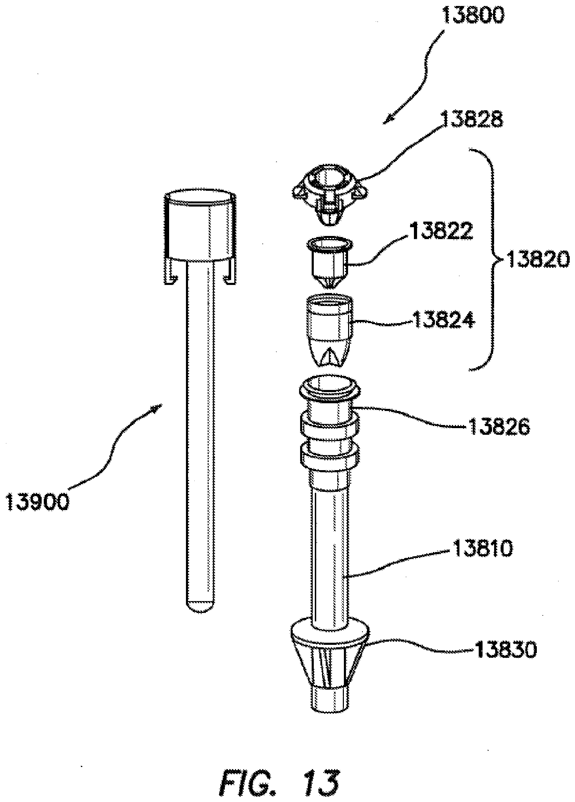

FIG. **13** is an exploded view of an embodiment of a trocar **13800** and optional obturator **13900,** which is a component of some embodiments of the access device system. In the illustrated embodiment, the obturator **13900** comprises a pointed, puncture tip **13910.** In embodiments in which the trocar **13800** and obturator **13900** are inserted through a gel pad **10530** rather than a body wall, potential damage to underlying tissue by contact with the tip **13910** is reduced because the gel pad **10530** serves as an artificial body wall that is spaced from the underlying tissue as discussed above. In other embodiments, the obturator tip **13910** has another shape, for example, blunt and/or bladeless, which, for example, reduces the likelihood of damage to other components of the access system, for example, a retraction sheath of a retractor.

The trocar **13800** comprises a proximal end, a distal end, and a longitudinal axis. The trocar **13800** comprises a cannula **13810** extending along the longitudinal axis. A trocar seal **13820** is disposed at the proximal end of the cannula **13810.** A retainer **13830** is disposed at the distal end or tip of the cannula **13810.** In the illustrated embodiment, the distal end or tip of the cannula **13810** is not angled. Other embodiments comprise an angled distal end or tip of the cannula **13810.** The illustrated embodiment of the trocar **13800** does not comprise an insufflation gas inlet. Consequently, the trocar **13800** is typically used in procedures in which a body cavity is not insufflated, or in which insufflation is provided through another device. Other embodiments of trocars are disclosed in U.S. Patent Application No. 11/677,994.

The cannula **13810** comprises an elongate, tubular cannula body **13812** dimensioned to accommodate an instrument or instruments received there through. In the illustrated embodiment, the cannula body **13812** is a substantially cylindrical tube, and extends through the gel pad **10530** in use. In the illustrated embodiment, the cannula body **13812** extends from the proximal end of the cannula **13810** to which the trocar seal **13820** is coupled, and which has a larger outer diameter than the cannula body **13812.**

In some embodiments, the cannula **13810** is comparatively short because the cannula body **13812** need only traverse the gel pad **10530** (FIG. **10A****),** which has a known and consistent thickness, rather than a body wall. Accordingly, some embodiments of the cannula body **13812** are not more than about 2-times longer, about 1.5-times longer, about 1.2-times longer, or about 1.1-times longer than the thickness of the gel pad. In some embodiments, the cannula body **13812** is less than about 20 mm, about 10 mm, or about 5 mm longer than the thickness of the gel pad. In some embodiments, the cannula body **13812** is about as long as the gel pad is thick. In other embodiments, the cannula body **13812** has a different length, for example, a length typical for a cannula used for traversing a body wall. Shorter length cannula bodies permit increased angular degrees of freedom for instruments passing there through. Embodiments of shorter cannula bodies also accommodate curved instruments. The cannula **13810** comprises any suitable biocompatible material. In some embodiments, the cannula **13810** comprises a flexible material.

The illustrated trocar seal **13820** comprises an instrument or septum seal **13822** and a zero seal **13824.** The instrument seal **13822** seals instruments passing there through, thereby maintaining pressurization in a body cavity such as pneumoperitoneum or pneumorectum. The zero seal **13824** provides a seal when no instrument passes through the trocar seal **13820.** The instrument seal **13822** and zero seal **13824** are received in a housing **13826** disposed at the proximal end of the cannula **13810** and secured therein by a seal cover **13828.**

The retainer **13830** is disposed at or near the distal end of the cannula **13810.** In the illustrated embodiment, the distal end of the cannula **13810** is generally perpendicular to the longitudinal axis thereof, or not angled. Other embodiments comprise an angled distal end or tip. In some embodiments, the retainer **13830** and cannula **13810** are integrated, while in other embodiments, the retainer **13830** and cannula **13810** are not integrated. In the illustrated embodiment, the proximal end of the retainer **13830** comprises a flange **13832** that is generally flat and perpendicular to the longitudinal axis, while the distal end is tapered, narrowing toward the distal end of the cannula **13810.** The flange **13832** reduces the likelihood of accidental or inadvertent removal of the trocar **13800** from the gel pad. Some embodiments of the proximal face of the flange **13832** comprise additional anchoring features, for example, at least one of barbs, spikes, ridges, texturing, and the like, which are configured to penetrate or bite into a distal face of the gel pad **10530.** In some embodiments, a diameter of the flange **13832** is from about 1.5 to about 2.5 times wider, or from about 2 to about 2.2 times wider than an outer diameter of the cannula body **13812.** Some embodiments of the trocar **13800** are 5-mm trocars, in which the outer diameter of the cannula body **13812** is from about 7 mm to about 8 mm.

The tapered end of the retainer **13830** facilitates insertion of the trocar **13800** through the gel pad, either by itself, or when assembled with the obturator **13900** extending there through. For example, in some embodiments, the retainer **13830** is inserted through a preformed opening in the gel pad **10530.** Because embodiments of the gel material of the gel pad **10530** have high elongation values, as discussed above, the retainer **13830** is insertable through a relatively small opening in the gel pad **10530,** yet resists inadvertent removal, as discussed above.

In some embodiments in which the retainer **13830** and cannula **13810** are not integrated, that is, are separate components, the retainer **13830** is secured to the cannula **13810** after the cannula **13810** is inserted through the gel pad. In some embodiments, the cannula **13810** and retainer **13830** are secured mechanically, for example, using latches, screw threads, clips, lock rings, ratchets, and the like. In some embodiments, the cannula **13810** and retainer **13830** are secured adhesively. In some embodiments, the position of the retainer **13830** is adjustable, for example, to accommodate gel pads of different thicknesses. In some embodiments, the cannula **13810** and/or retainer **13830** is secured to the gel pad, for example, adhesively.

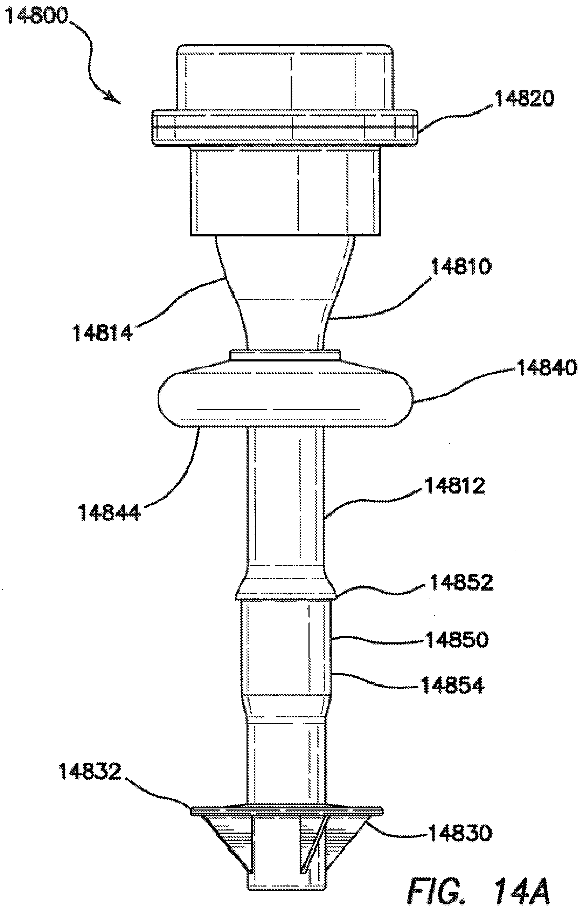

FIG. **14A** is a side view of another embodiment of a trocar **14800** that is suitable as a component of a single-port surgical access system described above, for example, comprising a gel pad **10530** and retractor. Some embodiments of the access system comprise a plurality of trocars **14800.** The trocar **14800** is generally similar to the trocar **13800** described above, and comprises a cannula **14810,** a trocar seal assembly **14820,** and a retainer **14830,** which are generally similar to the corresponding features described above. The illustrated embodiment of the trocar **14800** further comprises a bolster **14840** and a locking component **14850.** The illustrated embodiment of the cannula **14810** is also referred to as a "fixation cannula" as will become apparent from the discussion below.

In the illustrated embodiment, the bolster **14840** comprises a torus or doughnut. A cannula body **14812** extends through an opening in the bolster **14840.** A diameter of the opening of the bolster **14840** is sufficiently larger than an outer diameter of the cannula body **14812** to permit free movement along the cannula body **14812.** The illustrated embodiment of the bolster **14840** comprises a deformable material, for example, a polymer resin and/or elastomer, as will be described in greater detail below. Examples of suitable materials include rubber, natural rubber, synthetic rubber, polyisoprene, styrene-butadiene rubber, silicone rubber, ethylene-propylene copolymer, ethylene-propylene-diene monomer rubber, polybutadiene, polychloroprene, polyurethane, and the like. Some embodiments of the bolster **14840** comprise a lubricious layer or coating in an area or region that contacts the cannula **14810,** which facilitates movement along the cannula **14810.**

An outer diameter of some embodiments of the bolster **14840** is from about 0.8 to about 2 times, or from about 1 to about 1.5 times a diameter of a flange **14832** of the retainer **14830.** A thickness of the bolster is from about 3 mm (0.12 inch) to about 10 mm (0.4 inch), or from about 4 mm (0.16 inch) to about 6 mm (0.24 inch). In some embodiments, a distal face **14844** of the bolster is concave, thereby providing additional clamping or fixation force on the gel pad **10530,** as well as conforming to gel pads **10530** with different and/or non-uniform thicknesses. The particular dimensions of the bolster **14830** are selected based on the properties of the bolster material and the gel material, and the dimensions of the cannula body **14812,** the locking component **14850,** and the gel pad **10530.**

The locking component **14850** is disposed on the cannula body **14812** proximal of the retainer **14830,** and comprises a lip **14852** proximal of an enlarged section **14854.** The lip **14852** extends radially from the cannula body **14812** with a diameter greater than the diameter of the opening of the bolster **14840.** The elastomeric material of the bolster **14840** permits the bolster **14840** to be urged over and past the lip **14852.** In the illustrated embodiment, the lip **14852** comprises a ratchet dimensioned to facilitate the bolster **14840** sliding distally and to resist the bolster **14840** from sliding proximally. Also, in the illustrated embodiment, the lip **14852** is a continuous structure encircling the cannula body **14812.** In other embodiments, the lip **14852** comprises a plurality of structures disposed around the cannula body **14812.**

The enlarged section **14854** is generally cylindrical with a diameter that is about the same as or slightly larger than the diameter of the opening in the bolster **14840,** thereby frictionally engaging the bolster **14840** thereto. In the illustrated embodiment, the enlarged section **14854** is longer than a thickness of the bolster **14840.** In the illustrated embodiment, the enlarged section **14854** does not extend to or contact the flange **14832** of the retainer **14830,** thereby not reducing a surface area of a proximal face thereof, and thereby improving the removal resistance thereof. In other embodiments, the enlarged section **14854** extends to the retainer **14830.** Other embodiments do not comprise an enlarged section.

A distance between a distal end of the lip **14852** and a proximal face of the flange **14832** is equal to or slightly less than a sum of a thickness of the bolster **14840** and the gel pad **10530.** In some embodiments, the gel pad is from about 5 mm (about 0.4 inch) to about 30 mm (about 1.2 inch) thick, or from about 13 mm (about 0.5 inch) to about 25 mm (about 1 inch) thick.

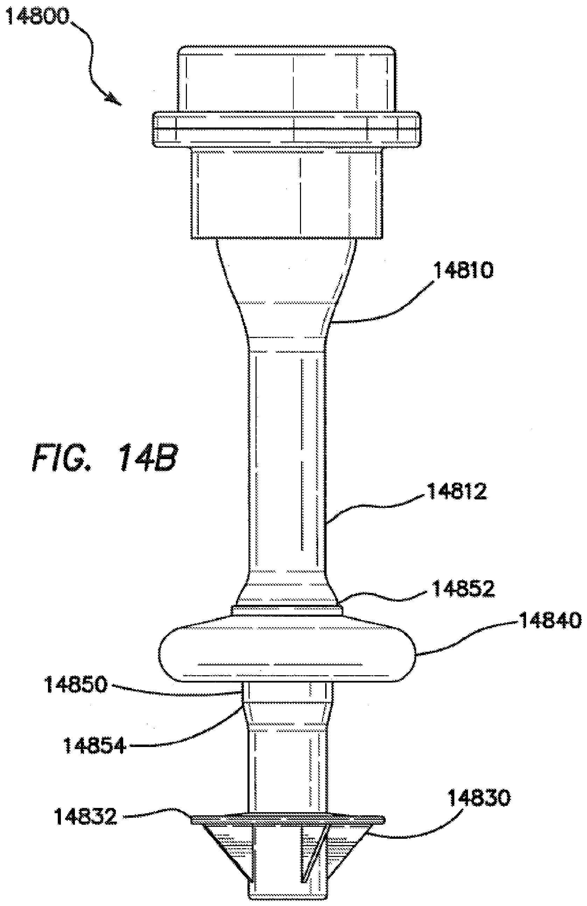

The trocar **14800** has at least two configurations: a first or insertion configuration illustrated in FIG. **14A****,** and a second or fixation configuration illustrated in FIG. **14B****.**

In an embodiment of a method for using the trocar **14800,** the trocar **14800** is placed in the insertion configuration in which the bolster **14840** is first positioned on the cannula body **14812.** The trocar **14800** is placed in the artificial body wall either before the artificial body wall is coupled to a patient's body and/or after coupling thereto.

In the embodiment illustrated in FIG. **14A****,** the bolster **14840** is positioned at the proximal end of the cannula body **14812,** where the bolster **14840** frictionally engages a distal portion of a cannula bell **14814,** which is an enlarged portion at the proximal end of the cannula **14810** to which the seal assembly **14820** couples.

The distal end of the trocar **14800** is positioned on, then the retainer **14830** inserted through an artificial body wall, for example, a gel pad **10530.** In some embodiments, an obturator **13900** (FIG. **13****)** is first inserted through the seal assembly **14820** at the proximal end of the trocar with the tip **13910** extending from the distal end thereof before this step. In other embodiments, an opening is first made in the artificial body wall using another instrument. In other embodiments, the distal end of the trocar **14800** is forced through the artificial body wall, generating an opening in the process.

The trocar **14800** is then converted into the fixation configuration illustrated in FIG. **14B** by sliding the bolster **14840** down the cannula body **14812,** and over the lip **14852** onto the enlarged section **14852.** In the illustrated configuration, the artificial body wall is captured and compressed between the flange **14830** of the retainer and the bolster **14840.** The lip **14852** locks the bolster **14840** in place, preventing it from moving proximally, thereby fixing or locking the trocar **14800** to the artificial body wall.

In the fixation configuration, the trocar **14800** fixed relative to a local portion of the artificial body wall to which it is engaged. As discussed above, however, embodiments of artificial body walls exhibit high elongations. Accordingly, the trocar **14800** is translatable and/or pivotable relative to an original position and orientation by deforming the artificial body wall.

In embodiments using an obturator **13910,** the obturator is withdrawn. The trocar **14800** serves as an access port for one or more instruments during a surgical procedure.

If desired, the trocar **14800** is removed from the artificial body wall, for example, by first disengaging the bolster **14840** from the locking component **14850,** then pulling the retainer **14830** from the artificial body wall. In some embodiments, the trocar **14800** and artificial body wall are not disengaged and are disposed of as a unit. In some embodiments, the bolster **14840** is not disengagable from the locking component **14850.**

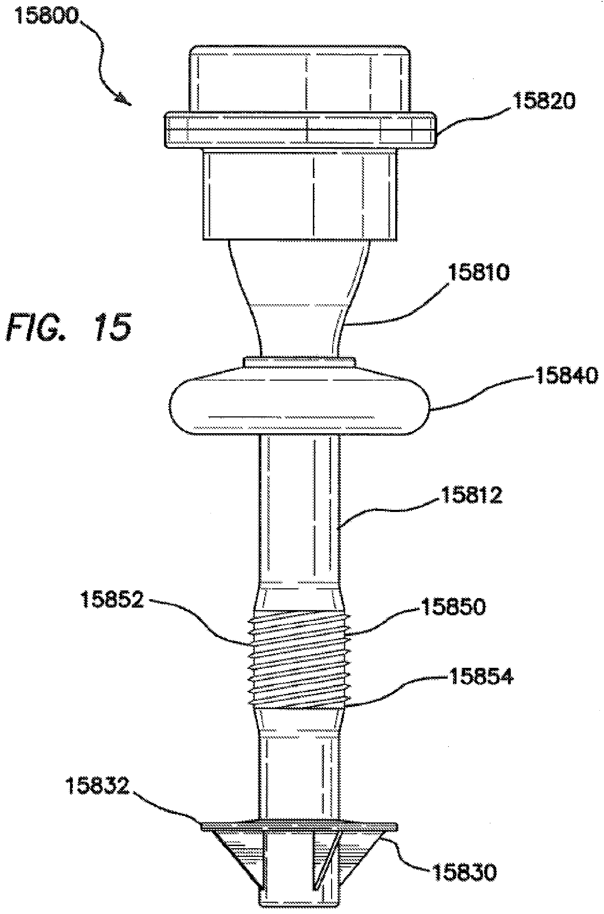

FIG. **15** is a side view of another embodiment of a retention trocar **15000,** which is generally similar to the embodiment illustrated in FIGS. **14A** and **14B** and described above. The trocar **15000** comprises an elongate, tubular cannula **15810** comprising a proximal end, a distal end, and a cannula body **15812;** a seal assembly **15820** coupled to the proximal end of the cannula **15810;** a retainer **15830** disposed at the distal end of the cannula **15810;** a bolster **14840** through which the cannula body **15812** extends; and a locking component **15850** disposed on the cannula body proximal of the retainer **15830.**

In the illustrated embodiment, the locking component **15850** comprises an enlarged section **15854** on which are disposed screw threads **15852.** The bolster **15840** comprises matching threads. Consequently, the bolster **15840** is threadably engagable to the locking component **15850.** The threading also permits adjusting the relative positions of the bolster **15840** and a flange **15832** of the retainer in the fixation configuration of the trocar **15800,** thereby permitting fixation to an artificial body wall with a non-uniform thickness and/or to artificial body walls of different thicknesses.

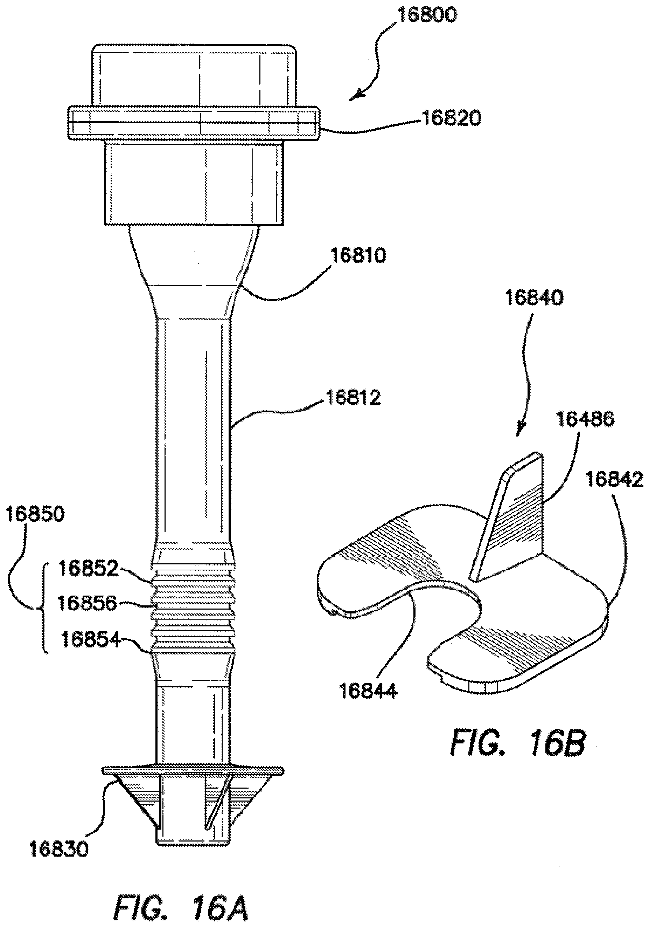

FIG. **16A** is a side view of another embodiment of a trocar **16800.** FIGS. **16B** is a perspective view of an embodiment of a bolster **16840** usable with the trocar **16800.** The combination of the trocar **16800** and bolster **16840** are generally similar to the embodiments of trocars illustrated in FIGS. **14A****,** **14B****,** and **15****.** The trocar **16800** comprises an elongate, tubular fixation cannula **16810** comprising a proximal end, a distal end, and a cannula body **16812;** a seal assembly **16820** coupled to the proximal end of the cannula **16810;** a retainer **16830** disposed at the distal end of the cannula **16810;** and a locking component **16850** disposed on the cannula body proximal of the retainer **16830.**

In the illustrated embodiment, the locking component **16850** comprises an enlarged section **16854** comprising a plurality of annular rings **16852** extending radially from the cannula body **16812,** which define a plurality of annular slots **16856.** In the illustrated embodiment, a proximal edge of each ring **16856** is beveled; however, some embodiments do not comprise a beveled edge.

FIG. **16B** illustrates an embodiment of a bolster **16840** in the form of a clip comprising a flattened body **16842** comprising a cut-out **16844** comprising a semicircular portion. The cut-out **16844** is dimensioned to engage the slots **16856.** A thickness of the body **16842** at the cut-out **16844** is also dimensioned to engage the slots **16856.** The bolster **16840** comprises a grip **16846** extending vertically from the body **16842,** which provides a user grip for installing and/or adjusting the bolster **16840.** In other embodiments, the cut-out **16844** has another shape, for example, polygonal, rectangular, a portion of a hexagon, and the like.

In use, the retainer **16830** of the trocar is inserted through an artificial body wall as discussed above, and fixed therein by engaging the bolster **16840** in a slot **16856** providing a desired fixation force. The degree of fixation is adjustable by selecting a different slot.

In some embodiments, the bolster cut-out **16844** engages a plurality of slots, thereby providing additional stability in the fixation configuration. Other embodiments comprise a bolster through with the cannula body **16812** extends, similar to the embodiments discussed above. In some of these embodiments, the locking component **16850** serves as a ratchet. The bolster comprises one or more pawls, which are optionally disengagable, thereby enhancing adjustability.

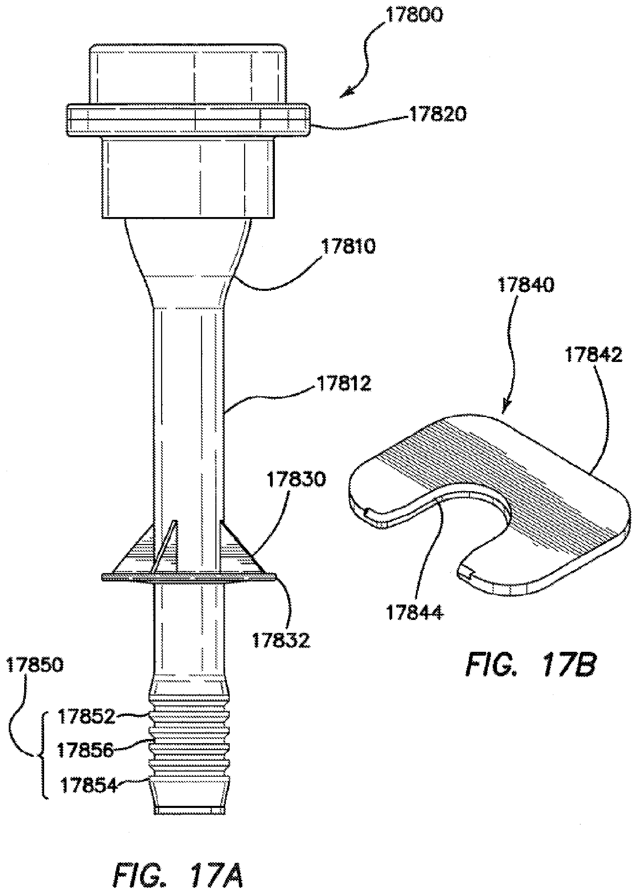

FIG. **17A** illustrates a side view of an embodiment of a trocar **17800** comprising a fixation cannula and FIG. **17B** is a perspective view of an embodiment of a bolster. The embodiments illustrated in FIGS. **17A** and **17B** are generally similar to the embodiments of trocars illustrated in FIGS. **14A-16B** and described above.

The trocar **17800** comprises an elongate, tubular fixation cannula **17810** comprising a proximal end, a distal end, and a cannula body **17812;** a seal assembly **17820** coupled to the proximal end of the cannula **17810;** a retainer **17830** disposed on the cannula body **17812;** and a locking component **17850** disposed at the distal end of the cannula **17810.** The illustrated embodiment of the trocar **17800** is similar to the embodiment illustrated in FIG. **16A** with the positions of the retainer **17830** and the locking component **17850** reversed. In the illustrated embodiment, a flange **17832** of the retainer faces distally.

The locking component **17850** comprises an enlarged section **17854** comprising a plurality of annular rings **17852** extending radially from the cannula body **17812,** which define a plurality of annular slots **17856.**

FIG. **17B** illustrates an embodiment of a bolster **17840** in the form of a clip comprising a flattened body **17842** comprising a cut-out **17844** comprising a semicircular portion. The cut-out **17844** is dimensioned to engage slots **17856** in the locking component. A thickness of the body **17842** at the cut-out **17844** is also dimensioned to engage the slots **17856.** The illustrated embodiment of the bolster does not comprise a grip; however, other embodiments comprise a grip.

In some embodiments for using the embodiment of the trocar **17800,** the cannula **17810** is fixed to an artificial body wall before the artificial body wall is coupled to a patient's body. For example, in some embodiments, one or more trocars **17800** are fixed on a gel pad **10530** (FIG. **10A****)** of a gel cap **10500** before the gel cap **10500** is coupled to a retractor **6100.**

With reference to those embodiments shown and described that are in accordance with the present invention, it will be understood by those of ordinary skill in the art that various changes in form and details may be made therein without departing from the scope of the invention as defined by the following claims.

## Claims

1. A surgical access port system adapted for performing surgical procedures at a natural orifice of a patient, the system comprising a retractor (6100) comprising:
an outer ring (6120), wherein the outer ring is configured to be disposed proximate the natural orifice of the patient;
a tubular body (6130) having a longitudinal axis, a proximal end and a distal end, wherein the proximal end of the tubular body is coupled to the outer ring; and
a flange (6111, 6112) disposed around the tubular body, the flange (6111, 6112) comprising a circular ridge (6109) circumscribing a channel (6107), **characterized in** the flange being cup-shaped with the channel (6107) having a width greater than a cross-sectional width of the outer ring (6120) and a depth greater than a wall thickness of the flange, such that the flange (6111, 6112) is adapted to secure the retractor (6100) within the natural orifice, with the flange (6111, 6112) in use under compression from walls of a vaginal canal, creating a seal to aid in maintenance of insufflation.

2. The surgical access port system of claim 1, wherein the proximal end of the tubular body comprises a funnel segment (6140).

3. The surgical access port system of claim 1, wherein the flange (6111) is disposed around the distal end of the tubular body (6130), and spaced from the outer ring (6120).

4. The surgical access port system of claim 1, wherein the flange (6112) is disposed around the proximal end of the tubular body (6130) near the outer ring (6120), the flange being disposed between the outer ring (6120) and the distal end of the tubular body (6130) with the distal end of the tubular body (6130) extended past the flange (6112) and positioned distally from the flange (6112); wherein the flange (6112) and the outer ring (6120) are configured to provide a distance between the flange (6112) and the outer ring (6120) to create a compressive force on tissue between the flange and the outer ring.

5. The surgical access port system of claim 1, further comprising:
a removable gel cap (10500), wherein the gel cap comprises a gel pad (10530) and a cap ring (10510) coupled with the gel pad, wherein the cap ring is engagable with the outer ring (6120);
a trocar (13800) having a cannula (13810) and a retainer (13830) disposed at a distal end of the cannula, the cannula does not comprise an insufflation gas inlet; and
an obturator (13900) having a pointed, puncture tip (13910), the trocar and obturator insertable through the gel pad.

6. The surgical access port system of claim 1, further comprising:
a frustoconical bolster (6113) disposed around the tubular body (6130), the bolster comprising a first diameter at a distal end and a second diameter at a proximal end, the second diameter being larger than the first diameter, wherein the bolster is adapted to externally occlude the natural orifice, the bolster being fixed around the proximal end of the tubular body; and
the flange (6111, 6112) disposed around the tubular body, distal to the bolster (6113).

7. The surgical access port system of claim 1, further comprising a mechanical balloon (6114) disposed around the tubular body (6130), the mechanical balloon comprising a series of arms (6105) disposed along the longitudinal axis of the tubular body, each arm having a proximal end attached to a first ring (6103) and a distal end attached to a second ring (6101), wherein the second ring is fixed to the tubular body; and wherein the flange is disposed around the tubular body, proximal to the mechanical balloon.

8. The surgical access port system of claim 4, wherein the distance between the flange and the outer ring is approximately 2.54 mm to 12.7mm, normally less than a thickness of tissues of a vaginal opening and arranged to create a compressive force on the tissues between the flange and the outer ring.

9. The surgical access port system of claim 1 , wherein the flange is semi-rigid, with a diameter from approximately 5.08 cm to 10.16 cm, wherein the cup-shaped flange is configured to be compressed from walls associated with the natural orifice thereby creating a seal that maintains insufflation, and wherein the flange provides flexibility configured to allow the flange to flex to accommodate different anatomies and maintain the seal with respect to the different anatomies.

10. The surgical access port system of any one of claims 1 to 7, further comprising one or more suture points (6160) disposed adjacent to the outer ring, wherein the one or more suture points and the flange are configured to prevent the retractor from dislodging once placed, and the outer ring includes a continuous annular groove (6122) formed therein in which a reinforcing member (6124) is disposed.

11. The surgical access port system of any one of claims 1 to 7, further comprising one or more suture points (6160) disposed adjacent to the outer ring, wherein the one or more suture points and the flange are configured to prevent the retractor from dislodging once placed, and the outer ring includes a plurality of noncontiguous recesses configured to receive one of a plurality of reinforcing members.

12. The surgical access port system of any one of claims 1 to 7, further comprising one or more suture points (6160) disposed adjacent to the outer ring, wherein the one or more suture points and the flange are configured to prevent the retractor from dislodging once placed, and the outer ring includes the outer ring includes two or more concentric generally annular grooves, which each receive a corresponding reinforcing member.

13. The surgical access port system of claim 2, wherein the tubular body and the flange is formed with a flexible material, and the funnel segment and the outer ring is formed with a rigid material different from the flexible material.

## Patentansprüche

1. Chirurgisches Zugangsanschlusssystem, das zur Durchführung von chirurgischen Eingriffen an einer natürlichen Körperöffnung eines Patienten geeignet ist, wobei das System einen Retraktor (6100) umfasst, der Folgendes umfasst:
einen äußeren Ring (6120), wobei der äußere Ring (6120) zur nahen Anordnung an der natürlichen Körperöffnung des Patienten konfiguriert ist;
einen rohrförmigen Körper (6130), der eine Längsachse, ein proximales Ende und ein distales Ende aufweist, wobei das proximale Ende des rohrförmigen Körpers mit dem äußeren Ring verbunden ist; und
einen Flansch (6111, 6112), der um den rohrförmigen Körper herum angeordnet ist, wobei der Flansch (6111, 6112) eine runde Riefe (6109) umfasst, die einen Kanal (6107) umgrenzt, **dadurch gekennzeichnet, dass** der Flansch kelchförmig ist, wobei der Kanal (6107) Folgendes aufweist: eine Breite, die größer ist als eine Querschnittsbreite des äußeren Rings (6120), und eine Tiefe, die größer ist als eine Wanddicke des Flansches, sodass der Flansch (6111, 6112) zur Befestigung des Retraktors (6100) innerhalb der natürlichen Körperöffnung geeignet ist, wobei der Flansch (6111, 6112) bei Verwendung unter Kompression von Wänden eines Vaginalkanals vorliegt, um eine Abdichtung zu erzeugen, die die Aufrechterhaltung von Insufflation unterstützt.

2. Chirurgisches Zugangsanschlusssystem nach Anspruch 1, wobei das proximale Ende des rohrförmigen Körpers einen Trichterabschnitt (6140) umfasst.

3. Chirurgisches Zugangsanschlusssystem nach Anspruch 1, wobei der Flansch (6111) um das distale Ende des rohrförmigen Körpers (6130) herum angeordnet ist und von dem äußeren Ring (6120) beabstandet ist.

4. Chirurgisches Zugangsanschlusssystem nach Anspruch 1, wobei der Flansch (6112) um das proximale Ende des rohrförmigen Körpers (6130) herum, nahe dem äußeren Ring (6120) angeordnet ist, wobei der Flansch zwischen dem äußeren Ring (6120) und dem distalen Ende des rohrförmigen Körpers (6130) angeordnet ist, wobei sich das distale Ende des rohrförmigen Körpers (6130) über den Flansch (6112) hinaus erstreckt und distal von dem Flansch (6112) positioniert ist; wobei der Flansch (6112) und der äußere Ring (6120) zur Bereitstellung eines Abstands zwischen dem Flansch (6112) und dem äußeren Ring (6120) konfiguriert sind, um eine Kompressionskraft auf Gewebe zwischen dem Flansch und dem äußeren Ring zu erzeugen.

5. Chirurgisches Zugangsanschlusssystem nach Anspruch 1, das weiter Folgendes umfasst:
eine entfernbare Gelkappe (10500), wobei die Gelkappe ein Gelkissen (10530) und einen Kappenring (10510), der mit dem Gelkissen verbunden ist, umfasst, wobei der Kappenring in den äußeren Ring (6120) eingreifen kann;
einen Trokar (13800), der eine Kanüle (13810) und einen Halter (13830), der an einem distalen Ende der Kanüle angeordnet ist, aufweist, wobei die Kanüle keinen Insufflationsgaseinlass umfasst; und
einen Obturator (13900), der eine spitze Einstechspitze (13910) aufweist, wobei der Trokar und Obturator durch das Gelkissen einführbar sind.

6. Chirurgisches Zugangsanschlusssystem nach Anspruch 1, das weiter Folgendes umfasst:
eine kegelstumpfförmige Verstärkung (6113), die um den rohrförmigen Körper (6130) herum angeordnet ist, wobei die Verstärkung einen ersten Durchmesser an einem distalen Ende und einen zweiten Durchmesser an einem proximalen Ende umfasst, wobei der zweite Durchmesser größer ist als der erste Durchmesser, wobei die Verstärkung zum externen Verschließen der natürlichen Körperöffnung geeignet ist, wobei die Verstärkung um das proximale Ende des rohrförmigen Körpers fixiert ist; und
der Flansch (6111, 6112) um den rohrförmigen Körper herum, distal zu der Verstärkung (6113) angeordnet ist.

7. Chirurgisches Zugangsanschlusssystem nach Anspruch 1, das weiter einen mechanischen Ballon (6114) umfasst, der um den rohrförmigen Körper (6130) herum angeordnet ist, wobei der mechanische Ballon eine Reihe von Armen (6105) umfasst, die entlang der Längsachse des rohrförmigen Körpers angeordnet sind, wobei jeder Arm Folgendes aufweist: ein proximales Ende, das an einem ersten Ring (6103) angebracht ist, und ein distales Ende, das an einem zweiten Ring (6101) angebracht ist, wobei der zweite Ring an dem rohrförmigen Körper fixiert ist; und wobei der Flansch um den rohrförmigen Körper herum, proximal zu dem mechanischen Ballon angeordnet ist.

8. Chirurgisches Zugangsanschlusssystem nach Anspruch 4, wobei der Abstand zwischen dem Flansch und dem äußeren Ring ungefähr 2,54 mm bis 12,7 mm beträgt, normalerweise weniger als eine Dicke von Geweben einer Vaginalöffnung, und zur Erzeugung einer Kompressionskraft auf die Gewebe zwischen dem Flansch und dem äußeren Ring angeordnet ist.

9. Chirurgisches Zugangsanschlusssystem nach Anspruch 1, wobei der Flansch halbstarr mit einem Durchmesser von ungefähr 5,08 cm bis 10,16 cm ist, wobei der kelchförmige Flansch konfiguriert ist, um von Wänden, die mit der natürlichen Körperöffnung in Verbindung stehen, komprimiert zu werden, wodurch eine Abdichtung erzeugt wird, die Insufflation aufrechterhält, und wobei der Flansch Flexibilität bereitstellt, die konfiguriert ist, um das Biegen des Flansches zu ermöglichen, um unterschiedlichen Anatomien zu entsprechen und die Abdichtung in Bezug auf die unterschiedlichen Anatomien aufrechtzuerhalten.

10. Chirurgisches Zugangsanschlusssystem nach einem der Ansprüche 1 bis 7, das weiter eine oder mehrere Nahtstellen (6160) umfasst, die benachbart zu dem äußeren Ring angeordnet sind, wobei die eine oder mehreren Nahtstellen und der Flansch konfiguriert sind, um zu verhindern, dass der Retraktor nach der Platzierung verschoben wird, und der äußere Ring eine fortlaufende ringförmige Rille (6122) einschließt, die darin ausgebildet, in der ein Verstärkungselement (6124) angeordnet ist.

11. Chirurgisches Zugangsanschlusssystem nach einem der Ansprüche 1 bis 7, das weiter eine oder mehrere Nahtstellen (6160) umfasst, die benachbart zu dem äußeren Ring angeordnet sind, wobei die eine oder mehreren Nahtstellen und der Flansch konfiguriert sind, um zu verhindern, dass der Retraktor nach der Platzierung verschoben wird, und der äußere Ring eine Vielzahl von nicht zusammenhängenden Aussparungen einschließt, die zur Aufnahme eines einer Vielzahl von Verstärkungselementen konfiguriert sind.

12. Chirurgisches Zugangsanschlusssystem nach einem der Ansprüche 1 bis 7, das weiter eine oder mehrere Nahtstellen (6160) umfasst, die benachbart zu dem äußeren Ring angeordnet sind, wobei die eine oder mehreren Nahtstellen und der Flansch konfiguriert sind, um zu verhindern, dass der Retraktor nach der Platzierung verschoben wird, und der äußere Ring einschließt der äußere Ring zwei oder mehr konzentrische, allgemein ringförmige Rillen einschließt, die jeweils ein entsprechendes Verstärkungselement aufnehmen.

13. Chirurgisches Zugangsanschlusssystem nach Anspruch 2, wobei der rohrförmige Körper und der Flansch aus einem flexiblen Material ausgebildet sind und der Trichterabschnitt und der äußere Ring aus einem starren Material ausgebildet sind, das sich von dem flexiblen Material unterscheidet.

## Revendications

1. Système de port d'accès chirurgical adapté pour effectuer des procédures chirurgicales à un orifice naturel d'un patient, le système comprenant un rétracteur (6100) comprenant :
un anneau externe (6120), dans lequel l'anneau externe est configuré pour être placé à proximité de l'orifice naturel du patient ;
un corps tubulaire (6130) ayant un axe longitudinal, une extrémité proximale et une extrémité distale, dans lequel l'extrémité proximale du corps tubulaire est couplée à l'anneau externe ; et
une bride (6111, 6112) placée autour du corps tubulaire, la bride (6111, 6112) comprenant une crête circulaire (6109) circonscrivant un canal (6107), **caractérisé en ce que** la bride étant en forme de coupe avec le canal (6107) ayant une largeur plus grande qu'une largeur en coupe transversale de l'anneau externe (6120) et une profondeur plus grande qu'une épaisseur de paroi de la bride, de sorte que la bride (6111, 6112) est adaptée pour fixer le rétracteur (6100) dans l'orifice naturel, avec la bride (6111, 6112) étant comprimée en emploi par les parois d'un canal vaginal, créant un joint d'étanchéité pour aider à maintenir l'insufflation.

2. Système de port d'accès chirurgical selon la revendication 1, dans lequel l'extrémité proximale du corps tubulaire comprend un segment en entonnoir (6140).

3. Système de port d'accès chirurgical selon la revendication 1, dans lequel la bride (6111) est placée autour de l'extrémité distale du corps tubulaire (6130) et espacée de l'anneau externe (6120).

4. Système de port d'accès chirurgical selon la revendication 1, dans lequel la bride (6112) est placée autour de l'extrémité proximale du corps tubulaire (6130) près de l'anneau externe (6120), la bride étant placée entre l'anneau externe (6120) et l'extrémité distale du corps tubulaire (6130) avec l'extrémité distale du corps tubulaire (6130) étendue au-delà de la bride (6112) et positionnée distalement de la bride (6112) ; dans lequel la bride (6112) et l'anneau externe (6120) sont configurés pour mettre une distance entre la bride (6112) et l'anneau externe (6120) afin de créer une force de compression sur un tissu entre la bride et l'anneau externe.

5. Système de port d'accès chirurgical selon la revendication 1, comprenant en outre :
un capuchon de gel amovible (10500) dans lequel le capuchon de gel comprend un coussinet de gel (10530) et une bague de capuchon (10510) couplée au coussinet de gel, dans lequel la bague de capuchon peut s'engager avec l'anneau externe (6120) ;
un trocart (13800) ayant une canule (13810) et un dispositif de retenue (13830) placé à une extrémité distale de la canule, la canule ne comprend pas d'entrée de gaz d'insufflation ; et
un obturateur (13900) ayant une pointe acérée perforante (13910), le trocart et l'obturateur pouvant être insérés à travers le coussinet de gel.

6. Système de port d'accès chirurgical selon la revendication 1, comprenant en outre :
un renfort tronconique (6113) placé autour du corps tubulaire (6130), le renfort comprenant un premier diamètre à une extrémité distale et un deuxième diamètre à une extrémité proximale, le deuxième diamètre étant plus grand que le premier diamètre, dans lequel le renfort est adapté pour occlure extérieurement l'orifice naturel, le renfort étant fixé autour de l'extrémité proximale du corps tubulaire ; et
la bride (6111, 6112) étant placée autour du corps tubulaire, distale au renfort (6113).

7. Système de port d'accès chirurgical selon la revendication 1, comprenant en outre un ballonnet mécanique (6114) placé autour du corps tubulaire (6130), le ballonnet mécanique comprenant une série de bras (6105) placés le long de l'axe longitudinal du corps tubulaire, chaque bras ayant une extrémité proximale attachée à un premier anneau (6103) et une extrémité distale attachée à un deuxième anneau (6101), dans lequel le deuxième anneau est fixé au corps tubulaire ; et dans lequel la bride est placée autour du corps tubulaire, proximale au ballonnet mécanique.

8. Système de port d'accès chirurgical selon la revendication 4, dans lequel la distance entre la bride et l'anneau externe est d'approximativement 2,54 mm à 12,7 mm, normalement moindre que l'épaisseur des tissus d'une ouverture vaginale et arrangée pour créer une force de compression sur les tissus entre la bride et l'anneau externe.

9. Système de port d'accès chirurgical selon la revendication 1, dans lequel la bride est semi-rigide avec un diamètre d'approximativement 5,08 cm à 10,16 cm, dans lequel la bride en forme de coupe est configurée pour être comprimée par des parois associées à l'orifice naturel créant ainsi un joint d'étanchéité qui maintient l'insufflation, et dans lequel la bride donne de la flexibilité configurée pour permettre à la bride de fléchir afin de s'adapter à différentes anatomies et maintenir le joint d'étanchéité par rapport aux différentes anatomies.

10. Système de port d'accès chirurgical selon l'une quelconque des revendications 1 à 7, comprenant en outre un ou plusieurs points de suture (6160) placés adjacents à l'anneau externe, dans lequel l'un ou plusieurs points de suture et la bride sont configurés pour empêcher le rétracteur de se déloger une fois mis en place, et l'anneau externe comprend une rainure annulaire continue (6122) formée dedans dans laquelle un élément de renforcement (6124) est placé.

11. Système de port d'accès chirurgical selon l'une quelconque des revendications 1 à 7, comprenant en outre un ou plusieurs points de suture (6160) placés adjacents à l'anneau externe, dans lequel l'un ou plusieurs points de suture et la bride sont configurés pour empêcher le rétracteur de se déloger une fois mis en place, et l'anneau externe comprend une pluralité d'évidements non contigus configurés pour recevoir l'un d'une pluralité d'éléments de renforcement.

12. Système de port d'accès chirurgical selon l'une quelconque des revendications 1 à 7, comprenant en outre un ou plusieurs points de suture (6160) placés adjacents à l'anneau externe, dans lequel l'un ou plusieurs points de suture et la bride sont configurés pour empêcher le rétracteur de se déloger une fois mis en place, et l'anneau externe comprend l'anneau externe comprend deux rainures concentriques ou plus généralement annulaires, qui reçoivent chacune un élément de renforcement correspondant.

13. Système de port d'accès chirurgical selon la revendication 2, dans lequel le corps tubulaire et la bride sont formés en un matériau flexible, et le segment en entonnoir et l'anneau externe sont formés en un matériau rigide différent du matériau flexible.
